Europäisches Patentamt

European Patent Office

Office européen des brevets

(19)

(11) **EP 1 541 083 A2**

(12) # EUROPEAN PATENT APPLICATION

(43) Date of publication:
**15.06.2005 Bulletin 2005/24**

(51) Int Cl.[7]: **A61B 5/05**

(21) Application number: **04028942.3**

(22) Date of filing: **07.12.2004**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **09.12.2003 JP 2003410888**
**09.12.2003 JP 2003410889**
**09.12.2003 JP 2003410890**
**09.12.2003 JP 2003410891**
**09.12.2003 JP 2003410892**

(71) Applicant: **Olympus Corporation**
**Shibuya-ku, Tokyo (JP)**

(72) Inventors:
• **Okamura, Toshiro**
  **Shibuya-ku, Tokyo (JP)**
• **Hatta, Shinji**
  **Shibuya-ku, Tokyo (JP)**
• **Taniguchi, Yuko**
  **Shibuya-ku, Tokyo (JP)**
• **Ito, Mitsuhiro**
  **Shibuya-ku, Tokyo (JP)**

(74) Representative: **von Hellfeld, Axel, Dr. Dipl.-Phys.**
**Wuesthoff & Wuesthoff**
**Patent- und Rechtsanwälte**
**Schweigerstrasse 2**
**81541 München (DE)**

(54) **Magnetic fluid detection device**

(57)     A magnetic fluid detection device (1) for detecting a magnetic fluid staying in a body to be examined includes an exciting unit for generating an exciting magnetic field to excite a magnetic fluid staying in a subject, a coil (22) for detecting a local distortion in magnetic field distribution occurring due to the magnetic fluid excited with the exciting magnetic field generated by the exciting unit, and a control unit (4) for signal-processing an output from the coil and informing the resultant signal magnitude.

FIG.4

TO CONTROL UNIT

EP 1 541 083 A2

**Description**

BACKGROUND OF THE INVENTION

1. Field of the Invention

The present invention relates to a magnetic fluid detection device for measuring the distribution of a magnetic fluid having magnetic properties infused in the vicinity of a tumor after a predetermined time period as the ground to identify a sentinel lymph node, which is a lymph node to which a tumor cell entering a lymph vessel from the primary seat of the tumor first reaches.

2. Description of the Related Art

In recent years, the finding ratio of early cancers has been enhanced. Thus, the resection operation of early cancers has been frequently carried out. In general, surgical operation on early cancers is carried out for the purpose of complete recovery. Therefore, in many cases, lesions and plural lymph nodes which exist around the lesions and to which the cancers may metastasize are resected. In the case of the surgical operation on early cancers, the resected lymph nodes are pathologically inspected after the operation. Thus, it is confirmed whether the cancers have metastasized to the lymph nodes or not. It is determined how to treat the patient after the operation, based on the pathological inspection results. In the operation stage, it is not known whether the cancers have metastasized to the lymph nodes or not. Therefore, for the operation on the early cancers, lymph nodes existing in the vicinity of the lesions are resected. This is a severe burden on the patient.

In recent years, it has been required that both of high QOL (Quality of Life) of a patient and his or her complete recovery by the cancer resection operation can be achieved. Accordingly, as one technique for satisfying such requirement, much attention has been given to Sentinel Node Navigation Surgery in which unnecessary resection of the lymph node to which cancers have not metastasized is prevented. Hereinafter, the sentinel node navigation surgery will be briefly explained.

Recent studies have revealed that caner does not metastasize to a lymph node at random, but metastasizes from the lesion to a lymph node via a lymph vessel according to a predetermined pattern. It is thought that cancer metastasizes to a sentinel lymph node whenever the metastasis to a lymph node occurs. The sentinel lymph node (SN) means a lymph node to which a cancer cell entering a lymph vessel from the primary seat of the cancer first reaches.

Therefore, in the operation on early cancer, the sentinel lymph node is found during the resection of the cancer, biopsy is carried out, and the pathological inspection is conducted. Thus, it is determined whether the cancer has metastasized to a lymph node or not. In the case where the cancer has not metastasized to the sentinel lymph node, it is not necessary to resect the remaining lymph nodes. In the case where the cancer has metastasized to the sentinel lymph node, plural lymph nodes in the vicinity of the lesion are resected in the operation on the early cancer, depending on the conditions of the metastasis.

Referring to the operation of early cancer, by carrying out the sentinel node navigation surgery, the resection of lymph nodes to which no cancer metastasizes can be prevented for a patient whom no cancer metastasizes to a lymph node. Thus, the burden on the patient can be reduced. The sentinel node navigation system is not limited to cancer of the breast or the like, and can be applied to laparotomy of digestive organs or the like, surgical operation using a laparoscope, and so forth.

Thus, for the sentinel node navigation surgery, it is earnestly required to develop a detection device which can detect a sentinel lymph node easily and accurately.

For example, Japanese Unexamined Patent Application Publication Nos. 2001-299676, 9-189770, 10-96782, U.S.P. No. 6,205,352, and so forth disclose the above-described detection device.

In recent years, SQUID flux meters using a superconducting quantum interference device (hereinafter, abbreviated to SQUID) have been applied in various fields. The SQUID device can detect a magnetic flux of which the strength is one billionth of that of the terrestrial magnetism with high sensitivity.

In recent years, regarding SQUID, a high temperature SQUID is applied for practical use, which can be used when it is cooled to a liquefied nitrogen temperature (77.3K: - 196°C).

A detection device using the high temperature SQUID has been proposed, e.g., as described in Journal of Japan Biomagnetism and Bioelectromagnetics, special number (vol. 15, No. 1, 2002, 17th, p.31-32 (Papers of Japan Biomagnetism and Bioelectromagnetics).

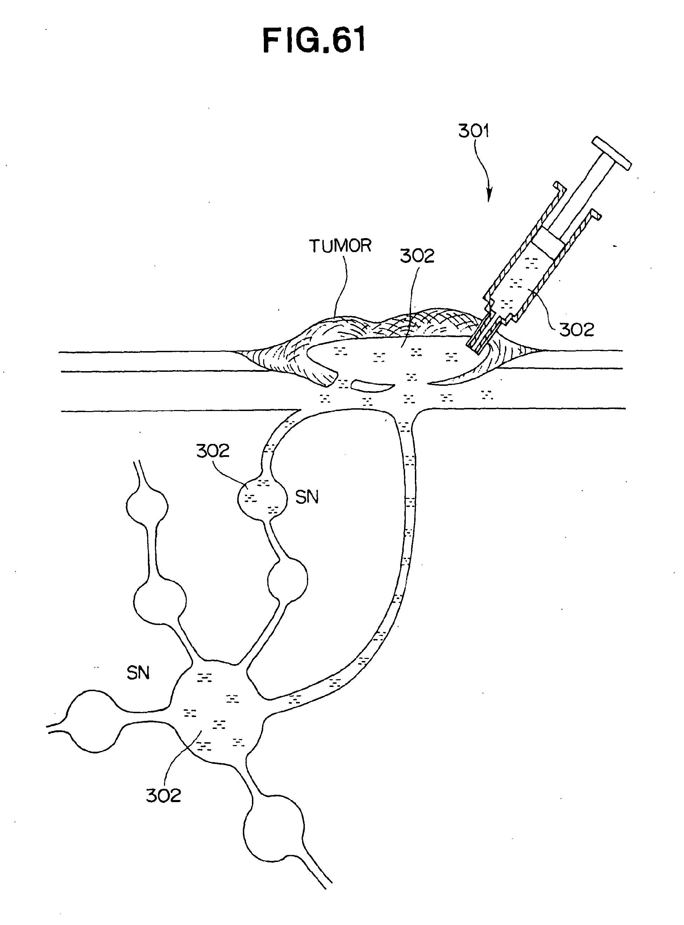

Referring to a detection method using the above-described high temperature superconducting SQUID, for example. a magnetic fluid 302 having magnetic properties as a tracer is infused locally in the vicinity of a tumor by use of an injector 301 such as a puncture needle or the like as shown in Fig. 61. The magnetic fluid 302 stays in a sentinel lymph node (SN) after a predetermined time period after the infusion.

According to the above-described detection device, the magnetic fluid is magnetized with an electromagnet having a large magnetic force. When the electromagnet is turned off, the weak residual magnetic field remaining in the magnetic fluid is detected by the SQUID. Thus, the sentinel lymph node can be detected.

The magnetic fluid has a very small particle size of several hundreds nm. Thus, the coercive force is small, and the strength of the residual magnetic field is very small. Thus, a magnetic sensor having a high sensitivity such as SQUID or the like is required.

However, the magnetic sensor using the high temperature superconducting SQUID (hereinafter, abbreviated to an SQUID magnetic sensor) is operated at a low temperature of about - 190°C. Thus, the sensor must be cooled with liquefied nitrogen. Thus, for the detection method using the high temperature superconducting SQUID, the size of the device is increased, and the manipulation property is deteriorated. Moreover, for the detection method using the high temperature superconducting SQUID, it is necessary to exchange the liquefied nitrogen every several hours. The running const is high. Furthermore, the detection method using the high temperature superconducting SQUID has problems in that the exchange of the liquefied nitrogen is a dangerous work, and so forth. Also, regarding to the detection method using the high temperature superconducting SQUID, the strength of the residual magnetic field remaining in the magnetic fluid is very small. Thus, the effects of magnetic noise from electrical devices of apparatuses are large, which requires magnetic shielding. Thus, the size of the detection device must be increased, and the cost becomes high.

On the other hand, regarding the above-described detection device, a device for detecting the magnetic fluid using plural magnetic sensors such as Hall elements, magnetic resistance elements, or the like has been proposed, e.g., as described in Japanese Unexamined Patent Application Publication No. 2003-128590.

According to the magnetic fluid detection device described in Japanese Unexamined Patent Application Publication No. 2003-128590, a exciting magnetic field is applied to the magnetic fluid by means of an exciting unit such as an exciting magnet or the like. The strength of the magnetic field component extending orthogonally to the exciting magnetic field and in the same magnetic field direction is detected by means of the plural magnetic sensors. One of the outputs from the plural magnetic sensors is subtracted from the other output, and the difference is signal-processed. Then, a local distortion in magnetic field distribution (spatial magnetic gradient) occurring due to the magnetic fluid excited with the exciting unit is detected.

However, the magnetic fluid detection device described in Japanese Unexamined Patent Application Publication No. 2003-128590 uses magnetic sensors such as Hall elements, magnetic resistance elements, or the like which give an output proportional to the strength of the magnetic field. The change of the magnetic field occurring due to the magnetic fluid is very small compared with the exciting magnetic field generated by the exciting unit. Therefore, if the outputs from the magnetic sensors are DC amplified, the outputs from the amplifiers become saturated. Thus, AC amplification must be carried out.

However, in the case where the magnetic sensors are AC coupled by means of a capacitor or the like, the signal change caused by the magnetic fluid is attenuated, unless the magnetic field is changed at a high speed by the magnetic fluid.

Therefore, according to the magnetic fluid detection device described in Japanese Unexamined Patent Application Publication No. 2003-128590, the outputs from the plural magnetic sensors are subtracted, so that a signal caused by the exciting magnetic field generated by the exciting unit is cancelled out, and the signal change caused by only the magnetic fluid is amplified.

Thus, since the magnetic fluid detection device described in Japanese Unexamined Patent Application Publication No. 2003-128590 uses the plural magnetic sensors, a detection portion containing the plural magnetic sensors increases in size. The manipulation property is deteriorated, and he cost increases.

Moreover, according to the magnetic fluid detection device described in Japanese Unexamined Patent Application Publication No. 2003-128590, if the strength of the exciting magnetic field generated by the exciting unit is increased to enhance the detection sensitivity for the magnetic fluid, the outputs of the magnetic sensors become saturated. Thus, the change of the magnetic field occurring due to the magnetic fluid cannot be detected.

OBJECT AND SUMMARY OF THE INVENTION

Accordingly, it is an object of the present invention to provide a magnetic fluid detection device of which the size is small, the manipulation property is superior, and the detection sensitivity for a magnetic fluid is high.

It is another object of the present invention to provide a magnetic fluid detection device of which the detection sensitivity is so high that even a trace amount of magnetic fluid can be detected.

A magnetic fluid detection device in accordance with the present invention which is useful for detecting a magnetic fluid staying in a subject comprises an exciting unit for generating an exciting magnetic field to excite a magnetic fluid staying in a subject, a coil for detecting a local distortion in magnetic field distribution occurring due to the magnetic fluid excited with the exciting magnetic field generated by the exciting unit, and a control unit for signal-processing an output from the coil and informing the resultant signal magnitude.

Moreover, a magnetic fluid detection device in accordance with the present invention which is useful for detecting a magnetic fluid staying in a subject comprises a probe comprising an exciting unit for generating an exciting magnetic field to excite a magnetic fluid staying in a subject, and a probe having a coil for detecting a local distortion in magnetic field distribution occurring due to the magnetic fluid excited with the exciting magnetic field generated by the exciting unit, and a control unit for signal-processing an output from the coil provided in the probe and informing the resultant signal magnitude.

Furthermore, a magnetic fluid detection device in accordance with the present invention which is useful for detecting a magnetic fluid staying in a subject comprises a probe comprising an exciting unit for generating an exciting magnetic field to excite a magnetic fluid staying in a subject, a coil for detecting a local distortion in magnetic field distribution occurring due to the magnetic fluid excited with the exciting magnetic field generated by the exciting unit, and a driving unit for integrally vibrating or revolving the exciting unit and the coil, and a control unit for controlling and driving the driving unit provided in the probe, signal-processing an output from the coil and informing the resultant signal magnitude.

BRIEF DESCRIPTION OF THE DRAWINGS

Fig. 1 illustrates a magnetic fluid detection device according to a first embodiment of the present invention;

Fig. 2 is a perspective view of the body of a probe from which a probe sheath in Fig. 1 is removed;

Fig. 3 is a schematic view of the probe body shown in Fig. 2;

Fig. 4 schematically shows the magnetic fluid detection device of Fig. 1;

Fig. 5 schematically shows a modification of the magnetic fluid detection device of Fig. 4;

Fig. 6 is a circuit block diagram of a control unit shown in Fig. 1;

Fig. 7 is a circuit block diagram showing a first modification of the control unit of Fig. 6;

Fig. 8 is a circuit block diagram showing a second modification of the control unit of Fig. 6;

Fig. 9 illustrates a coil having a large aperture;

Fig. 10 illustrates magnetic force lines passing through the aperture of the coil in Fig. 9 and magnetic noise;

Fig. 11 illustrates a coil having a small aperture;

Fig. 12 illustrates magnetic force lines passing through the aperture of the coil in Fig. 11;

Fig. 13 shows an enlargement of the magnetic force lines shown in Fig. 12;

Fig. 14 illustrates a top cover that is fixed to the probe sheath via an adjustment piece;

Fig. 15 illustrates the top cover that is fixed to the probe sheath by sliding, and is slightly pulled out from its position at which the top cover comes into contact with the body of a detection unit;

Fig. 16 illustrates the top cover that is fixed to the probe sheath by screwing;

Fig. 17 illustrates the top cover that is fixed to the probe sheath in the manner shown in Fig. 16, is slightly pulled out from its position at which the top cover comes into contact with the body of the detection unit, and then, is secured with an adhesive;

Fig. 18 illustrates an excited magnet and a motor magnet of which the polarities are arranged in the same direction;

Fig. 19 is a graph showing a relationship between the magnitude of a signal detected by a coil affected by a motor magnet and the distance from the coil to the magnetic fluid;

Fig. 20 schematically shows the magnetic fluid detection device having a correction magnet for correcting the magnetic field affected by the motor magnet;

Fig. 21 is a graph showing a relationship between the magnitude of a signal detected by a coil affected by the magnetic field of water and the distance of from the coil to the magnetic fluid;

Fig. 22 is a circuit block diagram for eliminating noise occurring due to the positional shift of a coil;

Fig. 23 is a graph showing a relationship between the signal magnitude detected by the coil and the frequency obtained in the circuit block diagram of Fig. 22;

Fig. 24 schematically shows a first modification of the magnetic fluid detection device which is configured so as not to be influenced with a motor magnet;

Fig. 25 schematically shows a second modification of the magnetic fluid detection device configured so as not to be affected by a motor magnet;

Fig. 26 schematically shows a third modification of the magnetic fluid detection device configured so as not to be affected by the motor magnet;

Fig. 27 schematically shows a fourth modification of the magnetic fluid detection device configured so as not to be affected by the motor magnet;

Fig. 28 illustrates a magnetic fluid detection device according to a second embodiment of the present invention;

Fig. 29 schematically shows a driving unit shown in Fig. 28;

Fig. 30 perspectively shows an eccentric cam shown in Fig. 29;

Fig. 31 is a schematic view of a first modification of a probe configured so that the detection rate is increased;

Fig. 32 is a schematic view of a modification of the drive unit in the first modification of the probe in Fig. 31;

Fig. 33 shows the appearance of a modification of the eccentric cam shown in Fig. 31;

Fig. 34 is a front view of the modification of the eccentric cam shown in Fig. 33;

Fig. 35 is a graph showing the time-dependent position of a vibration rod of the driving unit having one of the structures shown in Figs. 31 to 34;

Fig. 36 is a graph showing a relationship between the output signal from the coil and the time with which the position of the vibration rod varies as shown in the graph of Fig. 36.

Fig. 37 is a graph showing a relationship between the results obtained by signal-processing the output signal shown in of Fig. 36 and the time;

Fig. 38 is a graph showing a relationship between the output signal from the coil and the frequency obtained when other signal-processing is carried out;

Fig. 39 schematically shows a magnetic fluid detection device according to a third embodiment of the present invention;

Fig. 40 schematically shows a modification of the magnetic fluid detection device of Fig. 39;

Fig. 41 schematically shows the positions of two coils with respect to a motor;

Fig. 42 is a graph showing a relationship between the magnetic field strength of a motor magnet and the distance from the motor to the coil;

Fig. 43 is a schematic view of a detection unit body in which plural coils, an exciting magnet and preamplifiers are fixed by filling and hardening a resin;

Fig. 44 is a schematic view of magnetic force lines of the terrestrial magnetism extending perpendicularly across a coil;

Fig. 45 illustrates a case where the relative directions of the two coils with respect to the terrestrial magnetism of Fig. 44 are prevented from changing;

Fig. 46 illustrates a case where the relative directions of the two coils with respect to the terrestrial magnetism of Fig. 10 change;

Fig. 47 illustrates a magnetic fluid detection device according to a fourth embodiment of the present invention;

Fig. 48 schematically shows a driving unit of Fig. 47;

Fig. 49 shows the appearance of an eccentric cam of Fig. 48;

Fig. 50 schematically shows the distal-end side of a probe which can be vibrated in the right and left direction by causing the distal-end portion to swing in the right and left direction;

Fig. 51 shows a circuit configuration using an exciting electromagnet instead of an exciting magnet;

Fig. 52 is a graph showing a relationship between the magnitude of an output signal from one coil and the frequency, obtained in the circuit configuration of Fig. 51;

Fig. 53 is a graph showing a relationship between the magnitude of an output signal from the other coil and the frequency, obtained in the circuit configuration of Fig. 51;

Fig. 54 is a graph showing a signal magnitude of a difference signal obtained by subtracting the output from the coil in Fig. 53 from the output from the coil in Fig. 52;

Fig. 55 illustrates a magnetic fluid detection device according to a fifth embodiment of the present invention;

Fig. 56 schematically shows the magnetic fluid detection device in Fig. 55;

Fig. 57 schematically shows the structure of the distal-end revolution portion and its vicinity in Fig. 55;

Fig. 58 schematically shows a modification of the distal-end revolution portion and its vicinity in Fig. 57;

Fig. 59 illustrates a magnetic fluid detection device according to a sixth embodiment of the present invention;

Fig. 60 schematically shows a modification of the magnetic fluid detection device in Fig. 59; and

Fig. 61 illustrates the movement of a magnetic fluid caused when the magnetic fluid is used as a tracer.

DESCRIPTION OF THE PREFERRED EMBODIMENTS

Hereinafter, embodiments of the present invention will be described with reference to the drawings.

First Embodiment

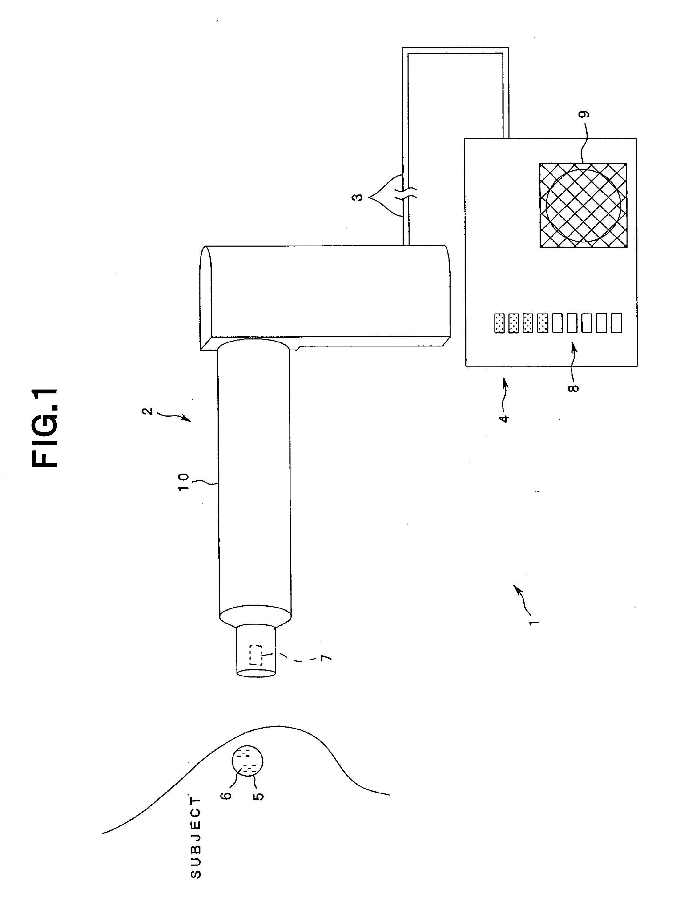

Figs. 1 to 27 show a magnetic fluid detection device according to a first embodiment of the present invention.

As shown in Fig. 1, a magnetic fluid detection device 1 comprises a probe 2, and a control unit 4 for controlling the probe 2 and connected to the probe 2 through a connecting cable 3.

The probe 2, when applied, is caused to contact the surface of a subject body from the outside of the body, is surgically inserted in an intracavity through a trocar, or is caused to contact the inside of the body after the surface of the body is surgically incised.

The probe 2 has a grip provided in the proximal-end side thereof so that the probe 2 can be easily gripped. Thus, the probe 2 has a pistol-like shape for easy handling. The probe 2 contains a detection unit 7 disposed in the distal-end side thereof. The detection unit 7 detects a magnetic fluid 6 staying in a sentinel lymph node in the subject. The detection unit 7 is provided with an exciting unit and a magnetic sensor, which will be described below.

The control unit 4 comprises a display 8 for displaying detection results obtained by the detection unit 7 and a speaker 9 for acoustically informing an operator of the detection results obtained by the detection unit 7, the display 8 and the speaker 9 being provided on a front panel. The display 8 comprises LEDs (Light Emitting Diode), LCD (Liquid Crystal Display), or the like. Thus, the control unit 4 can inform an operator of detection results of the magnetic fluid 6.

The probe 2 is covered with a probe sheath 10 made of a non-magnetic material. When the probe sheath 10 is removed from the probe 2, a probe body 11 is exposed. The probe 2 with the probe sheath 10 has a water-tight structure for use in an intracavity or the like.

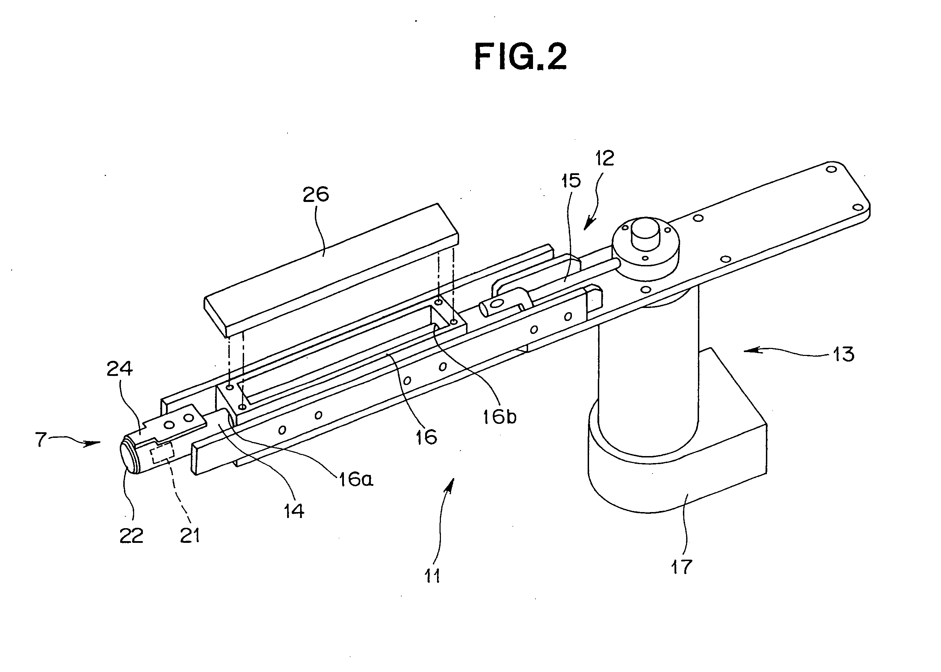

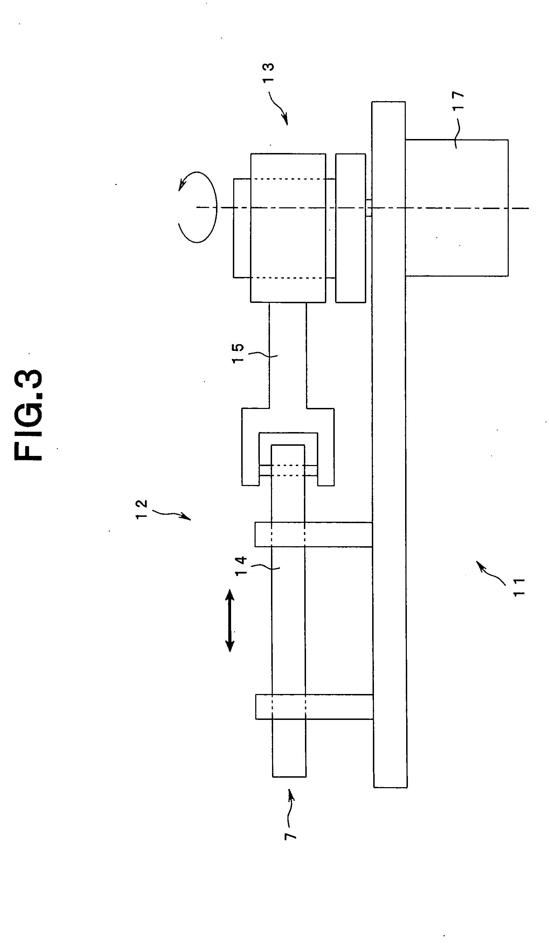

As shown in Figs. 2 and 3, the probe body 11 comprises a sliding unit 12 provided on the probe distal-end side, and a driving unit 13 provided on the probe proximal-end side.

The sliding unit 12 is made of a non-magnetic material. The sliding unit 12 comprises a vibration rod 14 and a connector 15.

The vibration rod 14 can be vibrated in the longitudinal axial direction. The distal-end side of the connector 15 is connected to the vibration rod 14, and the proximal-end side thereof is connected to the driving unit 13. The connector 15 is provided with a crank mechanism and so forth for transmitting vibration from the driving unit 13 to the vibration rod 14.

That is, the driving unit 13, the vibration rod 14, and the connector 15 constitute a vibration unit. The vibration rod 14 is formed to have a longer length than the connector 15 so that the detection unit 7 is separated far from the metallic part of the probe body 11.

The sliding unit 12 is provided with guides 16 at two positions, that is, on the distal-end side and the proximal-end side thereof. These guides 16 are formed so that the vibration rod 14 can be slid with being guided in the longitudinal axial direction.

The guide 16a on the distal-end side is fixed on the distal-end side of the vibration rod 14, while the guide 16b on the proximal-end side is fixed on the proximal-end side of the vibration rod 14.

The driving unit 13 contains a motor 17. The driving unit 13 converts the rotational motion of the motor 17 to the advancing and receding motion, which is transmitted to the connector 15.

The vibration rod 14 is slid on and guided by the guides 16a and 16b, and is vibrated in the longitudinal axial direction accompanying the vibration transmitted from the driving unit 13 via the connector 15.

If ball bearings are used between the vibration rod 14 and the guides 16, the ball bearings and the vibration rod 14, or the ball bearing and the driving unit 13 may adhere to each other, due to the generation of heat, which occurs due to the fact that the vibration distance is short.

According to this embodiment, the vibration rod 14 is vibrated while it is slid on and guided by the guides 16, as described above. Thus, the above-described adhesion, occurring due to the generation of heat, can be eliminated.

The driving unit 13 may contain a vibrator (not shown) instead of the motor 17 with which the vibration rod 14 is vibrated in the longitudinal axial direction.

The detection unit 7 is provided in the distal-end side of the vibration rod 14.

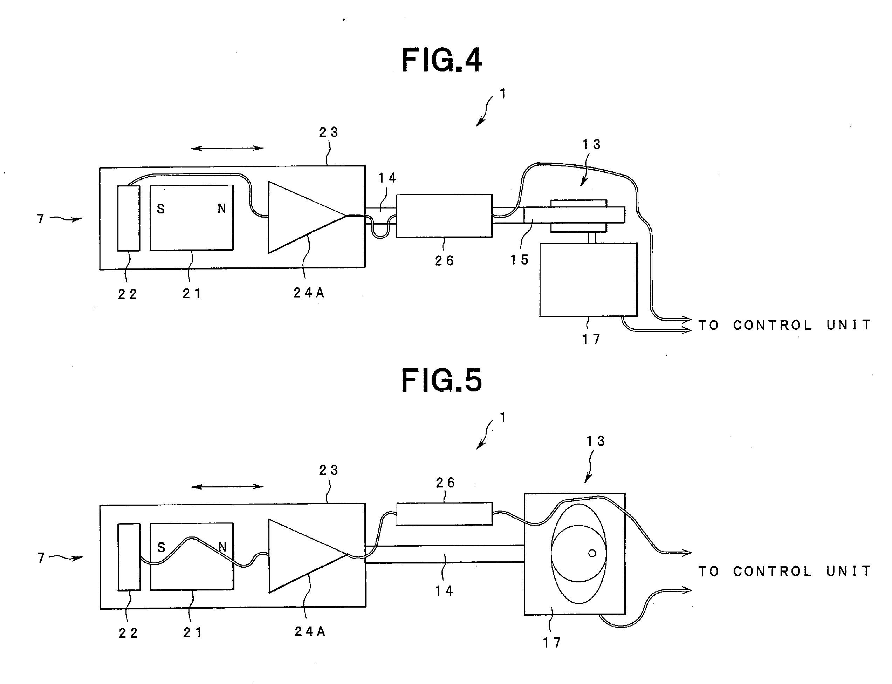

As shown in Fig. 4, the detection unit 7 comprises an exciting magnet 21 and a coil 22 provided in the body 23 of the detection unit 7. The exciting magnet 21 excites the magnetic fluid 6 staying in the subject. The exciting magnet 21 is a permanent magnet such as a neodymium magnet, a samarium - cobalt magnet, or the like. The coil 22 functions as a magnetic sensor for detecting a local distortion in magnetic field distribution (special magnetic gradient) which occurs due to the magnetic fluid 6 excited with the exciting magnet 21.

As shown in Fig. 5, the vibration rod 14 of the detection unit 7 may be connected directly to the motor 17 of the driving unit 13.

The coil 22 is provided in the distal-end side of the body 23 of the detection unit. The coil 22 is exposed on the distal-end side of the vibration rod 14. The exciting magnet 21 is arranged on the rear side of the coil 22.

In the detection unit 7, the exciting magnet 21 is vibrated in the longitudinal axial direction, accompanying the vibration in the longitudinal axial direction of the vibration rod 14. Thereby, the detection unit 7 generates an AC magnetic field as an exciting magnetic field, in response to the vibration frequency, so that the magnetic fluid 6 staying in the subject can be detected.

In general, the local distortion of the magnetic field distribution (special magnetic gradient), caused by the magnetic fluid 6, becomes larger in proportion to the strength of the exciting magnetic field (AC magnetic field), so that the magnetic fluid can be easily detected.

However, in case in which magnetic sensors such as Hall devices, magnetic resistance elements, or the like are used, and exciting magnets having a surface magnetic flux density of 0.1 T (tesla) or more is arranged, the output of a sensor is saturated, so that a change in magnetic field can not be detected. It is supposed that the distance between the magnetic fluid and the magnetic sensor is at least about 1 mm considering the thickness of a sheath.

In the case where magnetic sensors such as Hall devices, magnetic resistance elements, or the like, having a surface magnetic flux density of 0.1 T (tesla), are used, a magnetic fluid cannot be detected, unless the distance between the magnetic fluid and a magnetic sensor is 1 mm or less than 1 mm. If the distance is about 1 mm, the detection is almost impossible.

Accordingly, it is desirable that the surface magnetic flux density of the exciting magnet is not less than 0.1 T (tesla). However, in this case, Hall devices and magnetic resistance elements cannot be employed.

According to this embodiment, the coil 22 is used as a magnetic sensor. The electromotive voltage v by the coil 22 is generated according to the Faraday's electromagnetic induction law expressed by the following equation (1):

$$v = n\, \partial/\partial t \int_s H(t)ds \qquad (1)$$

in which n is the number of turns of the coil; and H(t) is a magnetic field.

In this case, the relative positional relationship between the exciting magnet 21 and the coil 22 is not varied. Therefore, only the static magnetic field is applied to the coil 22. The electromotive voltage v is zero according to the equation (1).

When the magnetic fluid 6 exists in the vicinity of the detection unit 7, the magnetic field distribution generated by the exciting magnet 21 is locally distorted due to the magnetic fluid 6 (spatial magnetic gradient), and the magnetic field applied to the coil 22 under vibration is changed. According to the equation (1), a voltage value proportional to the differential value of the change in magnetic field is output, as an electromotive voltage v, from the coil.

From the standpoint of the magnetic fluid 6, the exciting magnetic field generated by the exciting magnet 21 is an AC magnetic field.

As the magnetic force of the exciting magnet 21 is larger, the electromotive force v is larger, so that the detection sensitivity can be enhanced. The above-described permanent magnets such as neodymium magnets, samarium-cobalt magnets, or the like are small in size and have a large magnetic force. and hence, are suitable for use in the device of this embodiment. In the case of a neodymium magnet having a length of about 5 mm and a diameter φ of about 10 mm, the surface magnetic flux density is about 0.5 T (tesla).

Thus, according to this embodiment, the magnetic fluid 6 is excited by the AC magnetic field. The local distortion of the magnetic field distribution (spatial magnetic gradient) due to the magnetic fluid 6 is detected in the coil 22. Moreover, according to this embodiment, the vibration frequency component is detected based on the output of the coil 22, so that magnetic noise occurring due to the terrestrial magnetism, electrical devices or apparatuses, and so forth can be eliminated, as described below.

Moreover, the detection unit 7 is provided with a pre-amplification portion 24. The pre-amplification portion 24 contains a pre-amplifier 24A for amplifying the output from the coil 22.

That is, in the detection unit 7, the exciting magnet 21, the coil 22, and the pre-amplification portion 24 are integrally vibrated in the longitudinal axial direction, accompanying the vibration of the vibration rod 14 in the longitudinal axial direction.

Thus, according to this embodiment, a lead wire provided between the coil 22 and the pre-amplification portion 24 is prevented from being relatively vibrated, so that the detection unit 7 is not influenced with a change in contact resistance or the like. A lead wire provided between the pre-amplification portion 24 and a line driver 26 is vibrated. However, since the pre-amplification portion 24 amplifies a very small output from the coil 22, the change in magnitude of the output signal, occurring due to the contact resistance change or the like, is very small compared to the amplitude of the amplified output signal. Thus, the lead wire has no influence on the output signal.

Referring to the detection unit 7, the spaces between the coil 22, the exciting magnet 21, and the pre-amplification portion 24 are filled with a resin so as to be fixed to each other in the body 23 of the detection unit.

In the sliding unit 12, the line driver 26 for transmitting an output from the detection unit 7 to the control unit 4 is fixed near the vibration rod 14, separately from the vibration rod 14. That is, the line driver 26 is prevented from being vibrated. Thus, the line driver 26 being relatively heavy is not fixed to the vibration rod 14, and hence, the weight is prevented from adding to that of the vibration rod 14.

An output from the line driver 26 is transmitted to the control unit 4, in which the signal-processing is carried out.

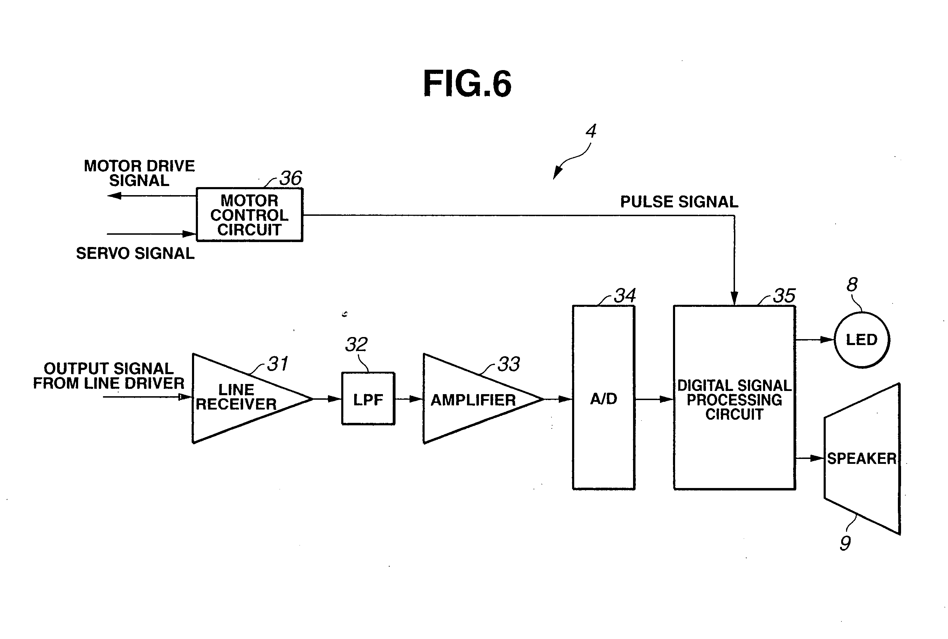

As shown in Fig. 6, the control unit 4 comprises a line receiver 31 for receiving an output from the line driver 26, a low-pass filter 32 (LPF) for eliminating a higher harmonic component from the output received by the line receiver 31 and passing the amplitude component, an amplifier 33 for amplifying a signal from LPF 32, an A/D converter 34 for A/D converting a signal from the amplifier 33, and a digital signal processing circuit 35 comprising, e.g., DSP (Digital Signal Processor) or the like for processing a digital signal A/D converted by the A/D converter 34 and driving the display or the speaker.

Moreover, the control unit 4 contains a motor control circuit 36 for controlling and driving the motor 17 of the driving unit 13. the motor control circuit 36 outputs a motor drive signal to drive the motor 17, and also, receives a servo signal from the motor 17 to carry out the feedback control, so that the rotational speed of the motor can be stabilized. Moreover, the motor control circuit 36 outputs a pulse signal synchronous with a rotational signal of the motor 17 to the digital signal processing circuit 35.

The digital signal processing circuit 35 demodulates the output signal from the coil 22 (the digital signal from the A/D converter 34), based on the pulse signal synchronous with the rotation of the motor from the motor control circuit 36, and detects the magnitude of the vibration frequency component, and drives the display 8 or the speaker 9 based on the detected signal magnitude.

For the demodulation, the pulse signal synchronous with the rotation of the motor is digitally multiplied by the output signal from the coil 22 (the digital signal from the A/D converter 34), or the output signal from the coil 22 is subjected to the high speed Fast Fourier Transform (FFT), and then, the frequency component having the vibration frequency determined based on the pulse signal synchronous with the rotation of the motor is determined.

When the rotational speed of the motor is stable, so that a further phasing component is not required, the output signal from the coil 22 (the digital signal from the A/D converter 34) can be demodulated while the vibration frequency is set at a constant value, and thus, the pulse signal synchronous with the rotation of the motor is not necessary.

In this case, the digital signal processing circuit 35 can change the luminance, flashing speed of LED of the display 8, the display state of an indicator composed of LEDs or the like, numerical display or indicator display on LCD, and so forth, in response to the magnitude of a detected signal.

Moreover, the digital signal processing circuit 35 can change the sound volume, the frequency, and the pulse train frequency of the speaker 9 in response to the magnitude of a detected signal.

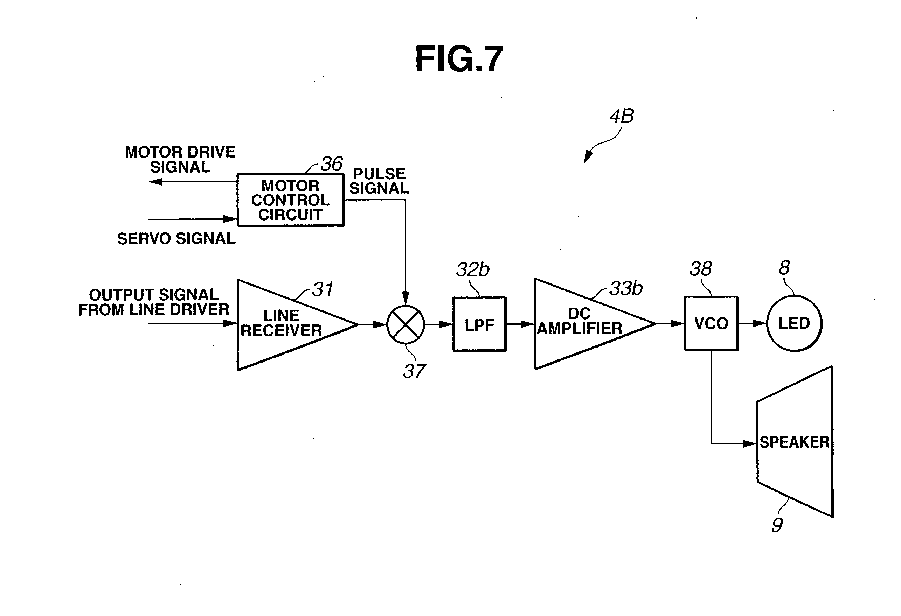

The control unit 4 shown in Fig. 6 is configured so as to process a digital signal. The control unit 4 may be configured so as to process an analog signal as shown in Fig. 7.

As shown in Fig. 7, a control unit 4B comprises the line receiver 31, a multiplier 37 for multiplying an output from the line receiver 31 by a pulse signal from the motor control circuit 36, LPF 32b for eliminating a higher harmonic component from the output from the multiplier 37 and passing a amplitude component, a DC amplifier 33b for amplifying an analog signal from LPF 32b, and a voltage controlled oscillator (VOC) 38 for driving the display 8 and the speaker 9 similarly to the digital signal processing circuit 35, in response to the strength of an analog signal (voltage) from the DC amplifier 33b.

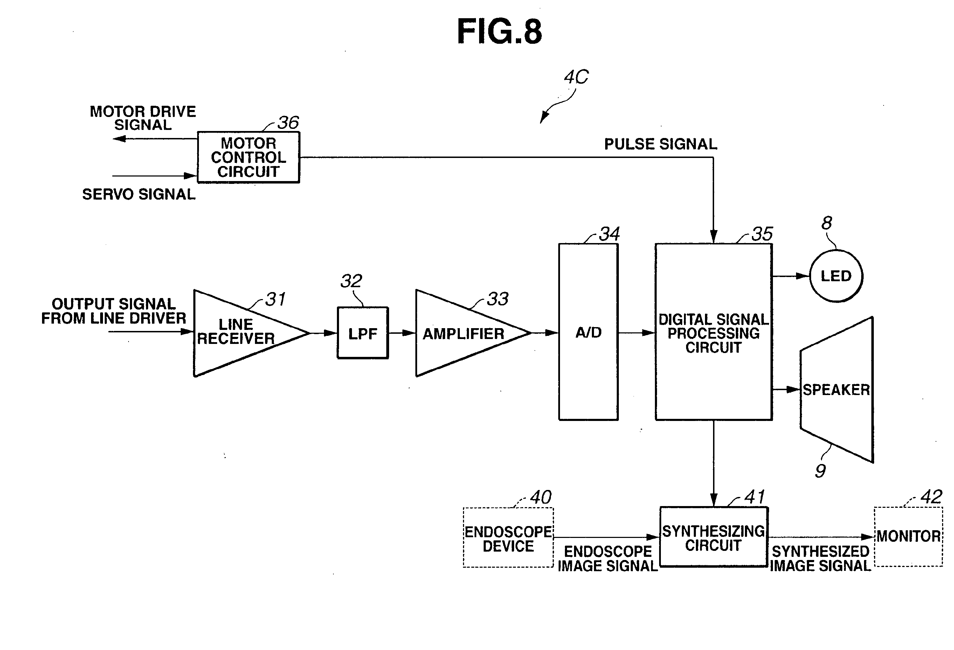

In the case in which the magnetic fluid detection device 1 is formed in combination with an endoscope device, a control unit may be configured so that detection results of the magnetic fluid 6 are displayed on a monitor on which an endoscope image is displayed, as shown in Fig. 8.

As shown in Fig. 8, a control unit 4C contains a synthesizing circuit 41 for synthesizing an endoscope image signal output from the endoscope device 40 with the detection results. The control unit 4C outputs the synthesized image signal from the synthesizing circuit 41 onto a monitor 42. Thus, the endoscope image and the detection results of the magnetic fluid 6 are displayed on the screen of the monitor.

As described above, the coil 22 is used as a magnetic sensor according to the present embodiment.

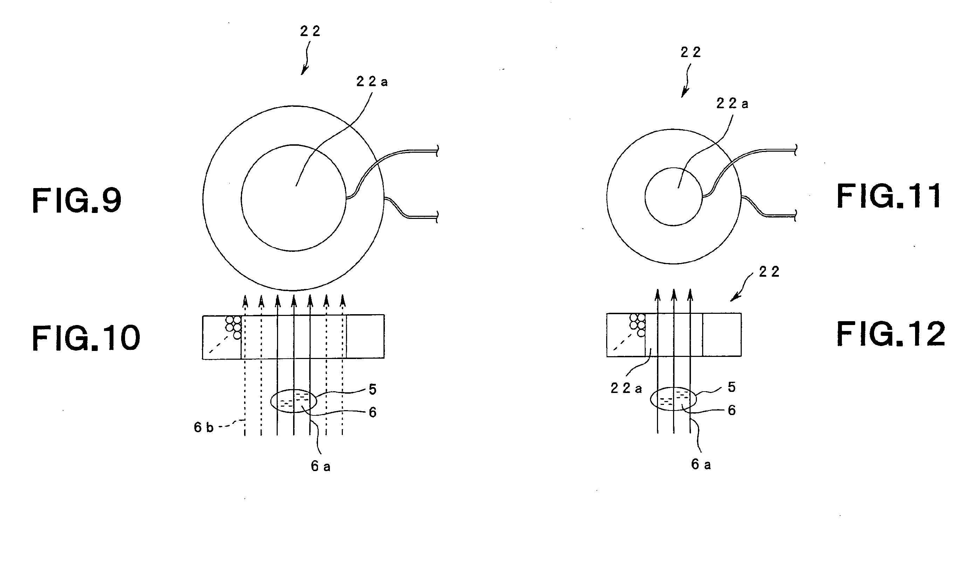

The coil 22 having a large aperture as shown in Figs. 9 and 10 has a large area in which magnetic noise 6b from electrical devices or apparatuses and so forth is detected in addition to the magnetic force lines 6a generated by the magnetic fluid 6. Thus, in the coil 22, the magnetic force lines 6a are covered with the magnetic noise 6b, and thus, the detection sensitivity is reduced. In general, the sizes of lymph nodes of a person are about 1 cm.

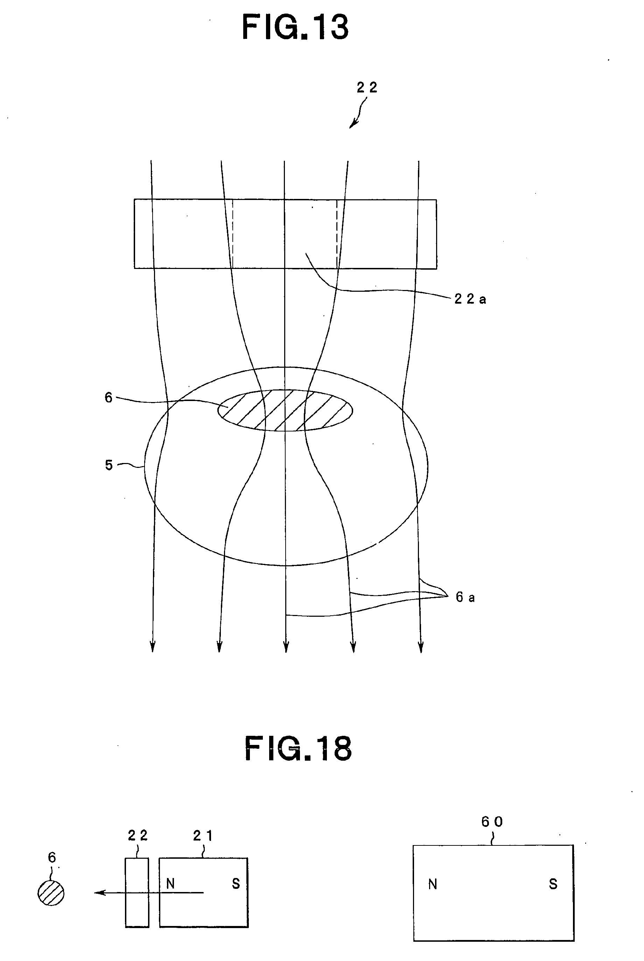

Therefore, according to this embodiment, the aperture 22a of the coil 22 is set at a size smaller than 1 cm, and thus, the aperture 22a has a size smaller than a lymph node as shown in Figs. 11 and 12. Thus, according to this embodiment, the coil 22 reduces as much as possible the area of detecting magnetic noise 6b from electrical devices or apparatuses, as shown in Fig. 13. The coil 22 can detect only the magnetic force lines 6a generated by the magnetic fluid 6.

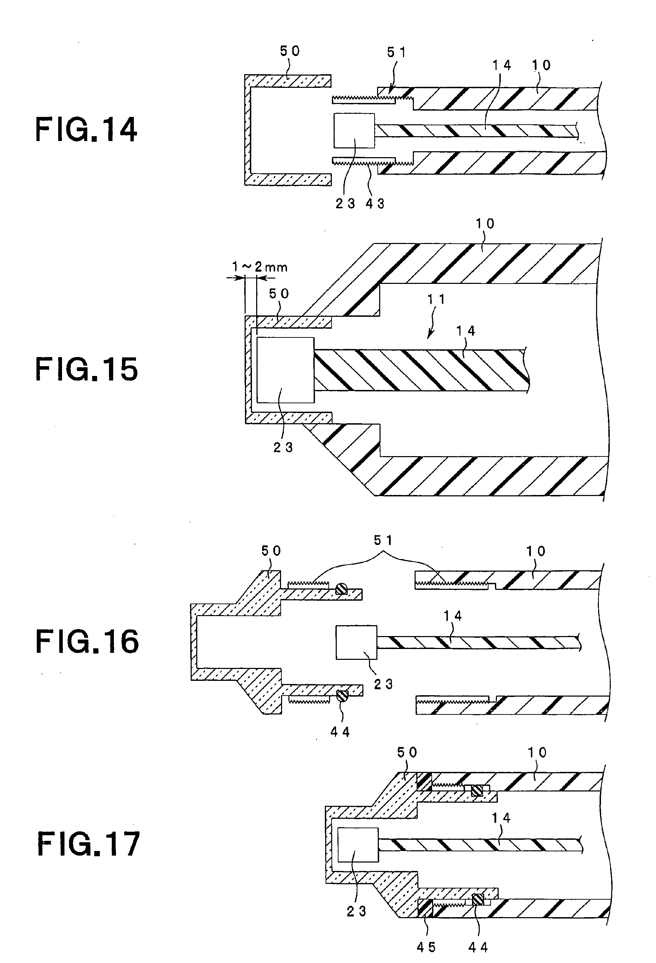

Moreover, according to this embodiment, the detection unit 7 is vibrated at an amplitude of about 1 mm to 2 mm in the longitudinal axial direction, which is caused by the vibration of the vibration rod 14 in the longitudinal axial direction. Thus, it is necessary to provide a space having a size of about 1 to 2 mm between the detection unit 7 and the probe sheath 10 so that the detection unit 7 can be vibrated (see Fig. 15). On the other hand, the thickness of the probe sheath 10 is in the range of about 0.5 to 1 mm.

The distance between the magnetic fluid and the coil 22 at which the magnetic fluid can be detected by the coil 22 is not more than about 5 mm. To increase the detection range as much as possible, according to this embodiment, a top cover 50 and the probe sheath 10, constituting a sheath, are formed separately from each other in such a manner that the distance between the top cover 50 and the body 23 of the detection unit can be adjusted.

Specifically, as shown in Fig. 14, the top cover 50 is fixed onto the probe sheath 10 via an adjusting piece 43. The adjusting piece 43 can be fixed to the probe sheath 10 through a screw portion 51 with a fine pitch. The adjusting piece 43 is fixed onto the probe sheath 10 at a position thereof where the end-face of the adjusting piece 43 slightly protrudes from the surface of the body 23 of the detection unit.

Then, the top cover 50 is placed onto the adjusting piece 43 and fixed thereto. Thus, the distance between the top cover 50 and the detection unit body 23 is minimized, and the detection range for the magnetic fluid 6 is maximized.

According to another method, the top cover 50 is slid on and attached to the probe sheath 10 as shown in Fig. 15. The top cover 50 is pulled out from the position at which the top cover 50 contacts with the detection unit body 23, and then is fixed. The method shown in Fig. 15 is simple compared to the method shown in Fig. 14, although the accuracy is slightly low.

According to still another method, as shown in Figs. 16 and 17, the top cover 50 is fixed onto the detection unit body 23 via the screw portion 51 with a fine pitch. In this case, the top cover 50 is provided with an O-ring 44 considering the water-tightness between them. The top cover 50 is slightly pulled out from the position at which the top cover 50 contacts with the detection unit body 23, and then is fixed using an adhesive 45.

The magnetic fluid detection device 1, formed as described above, detects the magnetic fluid 6 staying in a sentinel lymph node 5 of a subject to identify the sentinel lymph node 5.

First, an operator punctures the lower layer of a lesion of the subject with a puncture needle (not shown), and infuses the magnetic fluid 6 locally in the vicinity of the lesion. Then, the magnetic fluid 6 infused in the vicinity of the lesion is moved from the infusion position to a lymph vessel, reaches the sentinel lymph node 5 five or fifteen minutes after the infusion, and stays in the sentinel lymph node 5.

Then, the operator surgically inserts the probe 2 of the magnetic fluid detection device 1 into an intracavity, e.g., via a trocar (not shown), or is placed on the surface of the subject body from the outside of the body. The operator detects the magnetic fluid 6 staying in the sentinel lymph node 5 while the operator moves the distal end of the probe 2 in the vicinity of the lesion of the patient.

Then, the motor 17 of the driving unit 13 is driven while it is controlled with the motor control circuit 36 of the control unit 4. In the probe 2, the rotational motion of the motor 17 is converted to the advancing and receding motion, and the vibration is transmitted to the connector 15.

In the probe 2, the vibration rod 14 is vibrated in the longitudinal axial direction by the vibration transmitted from the driving unit 13 via the connector 15, while the vibration rod 14 is slid and guided by the guides 16a and 16b. Thereby, in the probe 2, the detection unit 7 is vibrated in the longitudinal axial direction. The exciting magnet 21 of the detection unit 7 is vibrated in the longitudinal axial direction. Thus, the probe 2 generates an AC magnetic field depending on the vibration frequency.

When the magnetic fluid 6 exists in the vicinity of the lesion of the patient, the AC magnetic field generated by the exciting magnet 21 excites the magnetic fluid 6 via the space in the vicinity of the probe. Then, the AC magnetic field is attracted or repelled in the vicinity of the magnetic fluid 6, so that the magnetic field distribution is locally distorted, and thus, the spatial gradient (magnetic flux density) of the magnetic field distribution changes. This local distortion of the magnetic field distribution (change of magnetic flux density), occurring due to the magnetic fluid 6, is detected by the coil 22.

In this case, the coil 22 can detect the local distortion of the magnetic field distribution occurring due to the magnetic fluid 6 without being influenced with the exciting magnetic field (the AC magnetic field from the standpoint of the magnetic fluid 6, and the static magnetic field from the standpoint of the coil 22), as described above. An output from the coil 22 is amplified by the pre-amplifier 24A, and is transmitted to the control unit 4 via the line driver 26.

In this case, in the detection unit 7, the pre-amplification portion 24, together with the exciting magnet 21 and the coil 22, is vibrated in the longitudinal axial direction, accompanying the vibration of the vibration rod 14 in the longitudinal axial direction. Thus, as described above, the lead wire between the coil 22 and the pre-amplification portion 24 is not vibrated, so that no change in the contact resistance or the like occurs, and hence, the detection unit 7 is not affected by such change.

The lead wire between the pre-amplification portion 24 and the line driver 26 is vibrated. However, the fine output from the coil 22 is amplified in the pre-amplification portion 24. Thus, even if the signal is varied by a change in contact resistance or the like, the change of the signal is slight compared to the signal magnitude after the amplification is carried out. Thus, such change of the contact resistance or the like does not exert an influence on the output signal substantially.

In the control unit 4, the line receiver 31 receives the output signal. LPF32 eliminates the higher harmonic component from the output from the line receiver 31, so that the amplitude component passes through LPF 32. The amplitude component is amplified by the amplifier 33 and is A/D converted by the A/D converter 34.

The digital signal processing circuit 35 demodulates the output signal from the coil 22 (the digital signal from the A/D converter 34), based on the pulse signal synchronous with the rotation of the motor output from the motor control circuit 36, detects the amplitude of the vibration frequency component, and drives the display 8 and the speaker 9 in response to the detected signal magnitude.

For the demodulation, the pulse signal synchronous with the rotation of the motor is digitally multiplied by the output signal from the coil 22 (the digital signal from the A/D converter 34), or the output signal from the coil 22 is subjected to the high speed Fourier transform, and then, the frequency component having the vibration frequency determined based on the pulse signal synchronous with the rotation of the motor is determined. When the rotational speed of the motor is stable, so that a further phasing component is not required, the output from the coil 22 (the digital signal from the A/D converter 34) can be demodulated while the vibration frequency is set at a constant value, and thus, the pulse signal synchronous with the rotation of the motor is not necessary.

The display 8 displays the local distortion of the magnetic field distribution using an indicator or figures. In this case, the display 8 displays the indicator or figures in such a manner that when the probe distal end approaches the magnetic fluid 6, the swing of the indicator becomes larger, or the numerical value becomes larger, and when the probe distal end becomes more distant from the magnetic fluid 6, the swing of the indicator becomes smaller or the numerical value becomes smaller.

The speaker 9 generates such a sound as corresponds to the local distortion of the magnetic field distribution (spatial magnetic gradient). In this case, when the probe distal end approaches the magnetic fluid 6, the sound emitted from the speaker 9 is larger. When the probe distal end becomes more distant from the magnetic fluid 6, the sound is smaller. The speaker 9 may generate a sound of which the frequency is proportional to the distance between the probe 2 and the magnetic fluid 6.

Thus, the magnetic fluid detection device 1 of the first embodiment has a small size, is superior in manipulation property, and can accurately detect the position of the magnetic fluid 6 staying in the sentinel lymph node 5 to identify the position of the sentinel lymph node 5.

Specifically, Feridekkusu (general name; ferumoxides), MnZn ferrite, $Fe_3O_4$ magnetite, or the like may be used for the magnetic fluid 6. When the particle sizes of these materials are small, the concentration of the magnetic fluid 6 becomes low when it stays in a lymph node. Thus, the force for distorting the magnetism is small. It is estimated that the relative magnetic permeability is substantially about 1.0001.

Accordingly, the output signal of the coil 22 is amplified so as to obtain a large gain. Thus, the output signal of the coil 22 is affected by a magnet used in the motor 17 for vibration disposed in the detection unit body 23 that is positioned farther from the coil 22 compared to the exciting magnet 21.

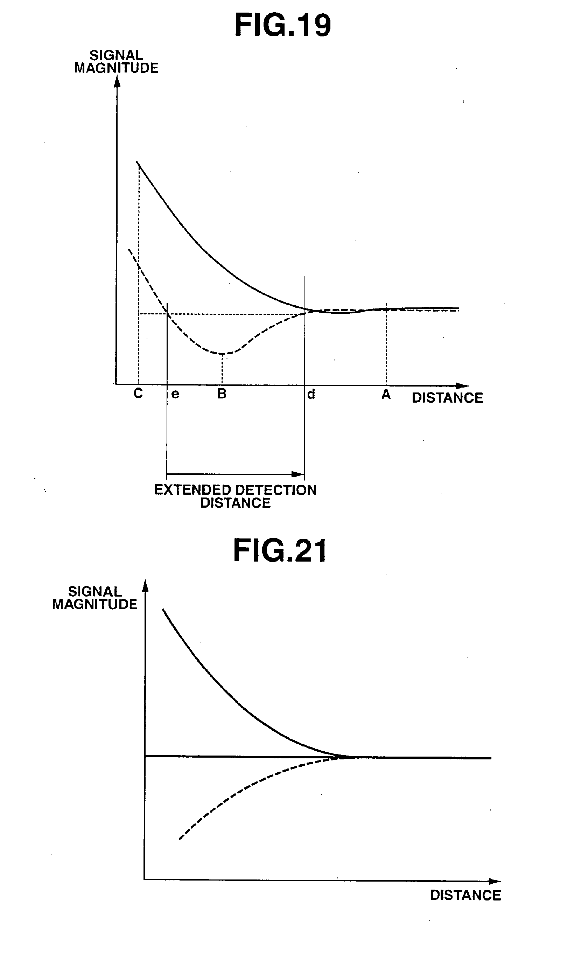

It is assumed that the device is assembled in such a manner that the magnetic poles of the exciting magnet 21 and those of a motor magnet 60 are arranged in the same direction, as shown in Fig. 18. When the coil 22 and the exciting magnet 21 approaches the magnetic fluid 6, the magnetic field is distorted as shown in Fig. 13. Thus, the magnetic field applied to the coil 22 increases.

At this time, the coil 22 becomes more distant from the motor magnet 60, and thus, the magnetic field from the motor magnet 60 applied to the coil 22 decreases. That is, the effect of the motor magnet 60 on the coil 22 (the magnetic field generated by the motor magnet 60 applied to the coil 22) decreases, while the effect of the magnetic fluid 6 on the coil 22 (the magnetic field generated by the magnetic fluid 6 applied to the coil 22) increases.

In this case, the magnitude of the signal output from the coil 22, obtained in the above-described case, is shown by a dotted line in Fig. 19.

When the magnetic fluid 6 exists (positions) far from the probe 2, the effect by the magnetic fluid 6 on the coil 22 is null, and thus, the output of the coil 22 is caused only by the effect of the motor magnet 60 (position A in Fig. 19).

When the magnetic fluid 6 exists at a position relatively near to the probe 2, the effect of the magnetic fluid 6 (the magnetic field generated by the magnetic fluid 6) and the effect of the motor magnet 60 (the magnetic field generated by the motor magnet 60) become equal. Thus, the magnitude of the signal output from the coil 22 approaches zero (position B in Fig. 19).

When the magnetic fluid 6 exists still nearer to the probe 2, the effect by the magnetic fluid 6 is larger than that by the magnetic poles of the motor magnet 60, and the magnitude of the signal output from the coil 22 becomes large (the position C in Fig. 19).

In the above-described case, in the range from position e to position d in Fig. 19, the magnitude of the signal output from the coil 22 is smaller than that obtained when no magnetic fluid 6 exists. Therefore, it cannot be determined whether the magnetic fluid exists or not. Substantially, the determination is possible in the range to the position e in Fig. 19.

It is assumed that the magnetic poles of the exciting magnet 21 and those of the motor magnet 60 are arranged in the directions opposite to each other. When the coil 22 and the exciting magnet 21 approach the magnetic fluid 6, the magnetic field applied to the coil 22 becomes large.

At this time, the magnetic field from the motor magnet 60 becomes small. However, since the polarities of the exciting magnet 21 and those of the motor magnet 60 are opposite to each other, the magnetic fields generated by the exciting magnet 21 and the motor magnet 60 are intensified by each other. Accordingly, the magnitude of the signal output from the coil 22 is shown by solid line in Fig. 19.

In the above-described case, the magnitude of the signal output from the coil 22 does not become lower than that obtained when no magnetic fluid exists. Therefore, the signal from the coil 22 can be measured in the range to the position d in Fig. 19.

Thus, the detection distance of the magnetic fluid can be maximized by assembling the device in such a manner that the magnetic poles of the exciting magnet 21 and those of the motor magnet 60 are arranged in directions opposite to each other.

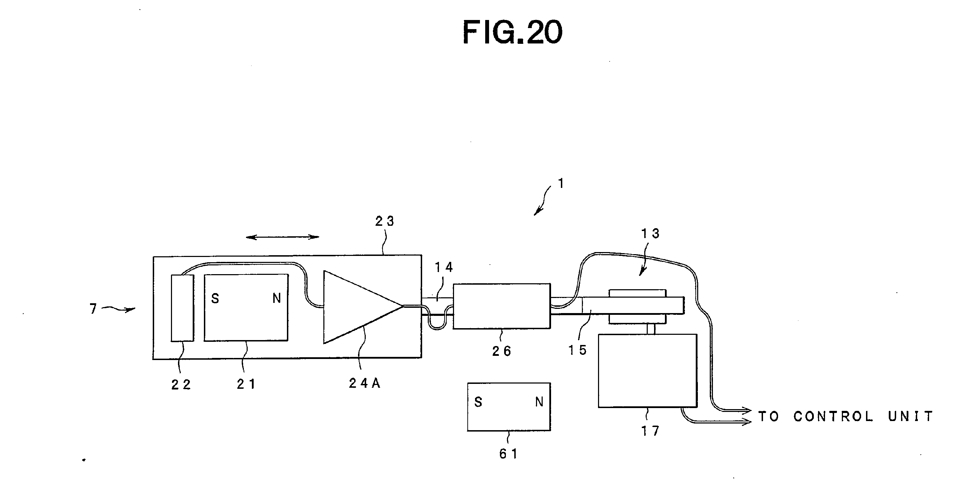

Moreover, as shown in Fig. 20, a correcting magnet 61 may be arranged in such a direction as to cancel out the magnetic field generated by the motor magnet 60.

The probe 2, when it is used in a living body, is affected by water contained in the living body. The relative magnetic permeability of water is about 0.999991. The difference in relative magnetic permeability between air and water is 0.00001. The difference is in the range of one fifth to one tenth of the difference in relative magnetic permeability between the magnetic fluid and water. In the case where the concentration of the magnetic fluid is low, the relative magnetic permeability of the magnetic flux is nearly equal to that of air, and no significant difference between them is found.

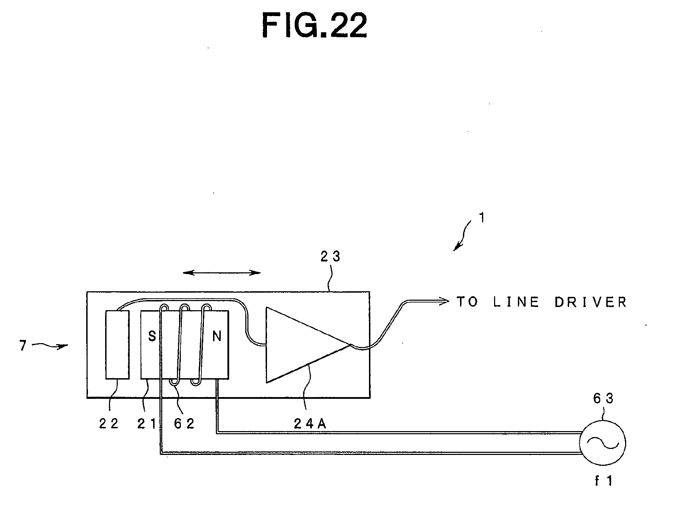

In this case, when the magnetic force of the correcting magnet 61 is increased, the coil 22 exhibits a large output signal magnitude, as shown in Fig. 21, due to the effect of the magnetic field of the correcting magnet 61, even when the probe 2 is set in the air.

It is assumed that the magnetic poles of the correcting magnet 61 and those of the exciting magnet 21 are arranged in directions opposite to each other. In this case, when the probe 2 approaches the magnetic fluid, the magnetic field applied from the magnetic fluid to the coil 22 increases, while the magnetic field applied from the correcting magnet 61 to the coil 22 decreases. Since the magnetic poles of the correcting magnet 61 and those of the exciting magnet 21 are arranged in the opposite directions, the magnetic fields are intensified by each other. Thus, the magnitude of the signal output from the coil 22 is shown by a solid line in Fig. 21.

When the probe 2 approaches water, the strength of the magnetic field applied to the coil 22 decreases, since the relative magnetic permeability of water is less than 1. Also, the strength of the magnetic field applied from the correcting magnet to the coil 22 also decreases. The magnetic fields from the correcting magnet 61 and the exciting magnet 21 are directed so as to be cancelled out by each other, since the magnetic poles of them are arranged in the opposite directions. Accordingly, the magnitude of the signal output from the coil 22 is shown by a dotted line in Fig. 21.

The change of the signal caused by water and the magnetic fluid may be reversed by increasing the magnetic force of the correcting magnet 61, thereby enabling detection of the magnetic fluid in the living body.

If the coil 22 is relatively shifted from the exciting magnet 21 due to the vibration in the longitudinal axial direction in the detection unit 7, noise will be generated due to the positional shift and detected.

In order to eliminate the noise due to the positional shift of the coil 22, the detection unit is configured as shown in Fig. 22.

In the detection unit 7 shown in Fig. 22, a correcting coil 62 for detecting a positional shift of the coil 22 is wound around the exciting magnet 21, and Ac current is supplied from the an AC electric source to the correcting coil 62.

The strength of an AC magnetic field f1 generated by the correcting coil 62 is set at such a low value that the AC magnetic field f1 can affect the coil 22 only, not exerting an influence onto the magnetic fluid 6.

When the detection unit 7 is vibrated in the longitudinal axial direction, noise generated by the positional shift of the coil 22, in addition to the local distortion of the magnetic field distribution (spatial magnetic gradient), generated due to the magnetic fluid 6, is superposed on the output from the coil 22.

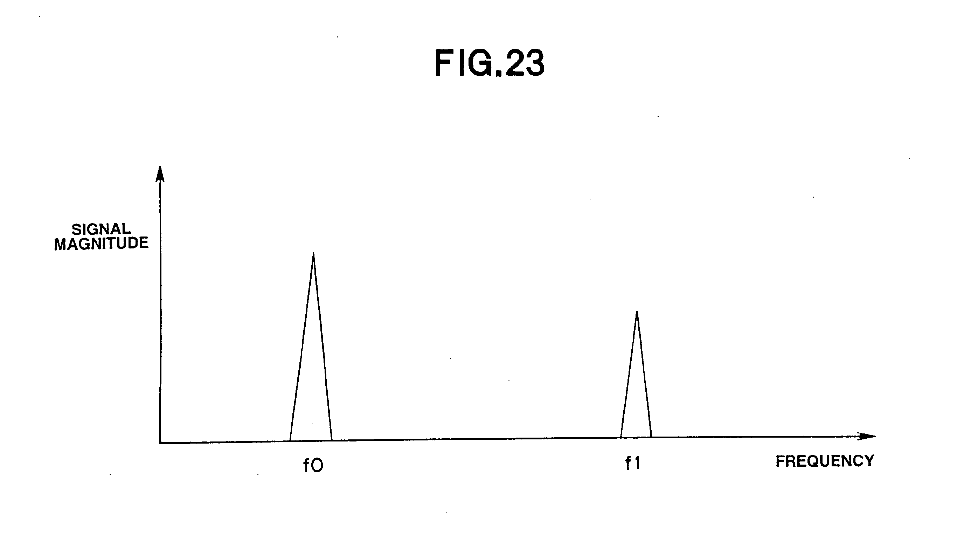

In particular, the noise by the positional shift of the coil 22, in addition to the local distortion of the magnetic field distribution (spatial magnetic gradient) occurring due to the magnetic fluid 6, is superposed on the magnitude of the signal detected by the coil 22 in the vicinity of a vibration frequency f0 shown in Fig. 23. The noise occurring due to the positional shift of the coil 22 is also superposed on the AC magnetic field f1 of the correcting magnet 61. The effect of the magnetic fluid 6 is not superposed on the AC magnetic field f1.

Thus, the strength of the AC magnetic field f1 multiplied by a predetermined coefficient is subtracted from the magnitude of the signal in the vicinity of the vibration frequency f0. Thereby, the noise occurring due to the positional shift of the coil 22 can be eliminated.

Thus, the noise generated by the positional shift of the coil 22 can be eliminated by the subtraction-processing of the output from the coil 22 in the control unit 4.

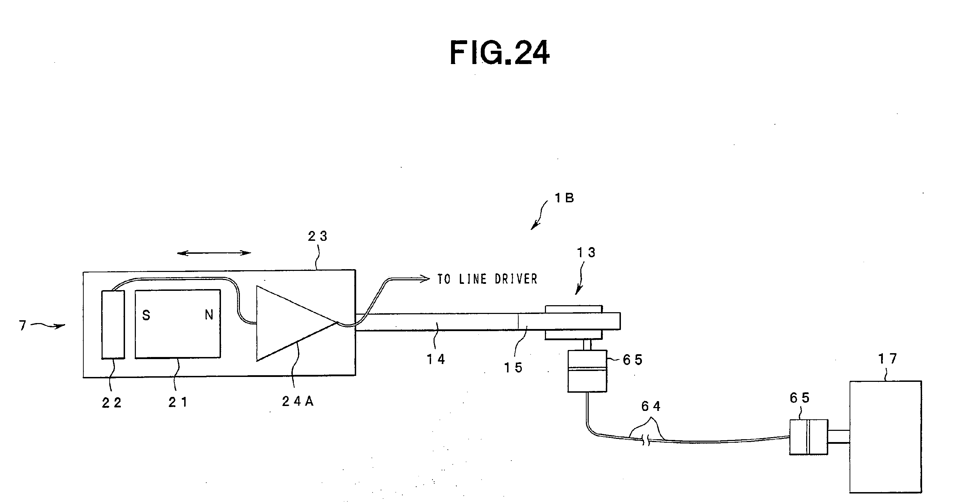

The magnetic fluid detection device 1 may be configured as shown in Figs. 24 to 27, so that affects by the motor magnet 60 can be eliminated.

In a magnetic fluid detection device 1B shown in Fig. 24, the motor 17 is disposed far from the probe side using a flexible shaft 64. Couplers 65 are used for the connection of the connector 15 to the flexible shaft 64 and for the connection of the flexible shaft 64 to the motor 17.

Thus, in the magnetic fluid detection device 1B, the rotational motion of the motor 17 is transmitted via the flexible shaft 64 and the couplers 65, and the transmitted rotational motion of the motor 17 is converted to the advancing and receding motion. Then, the vibration is transmitted to the connector 15.

Thus, in the magnetic fluid detection device 1B, the detection unit 7 is positioned far from the probe side, and hence, the detection unit 7 is prevented from being affected by the motor magnet 60.

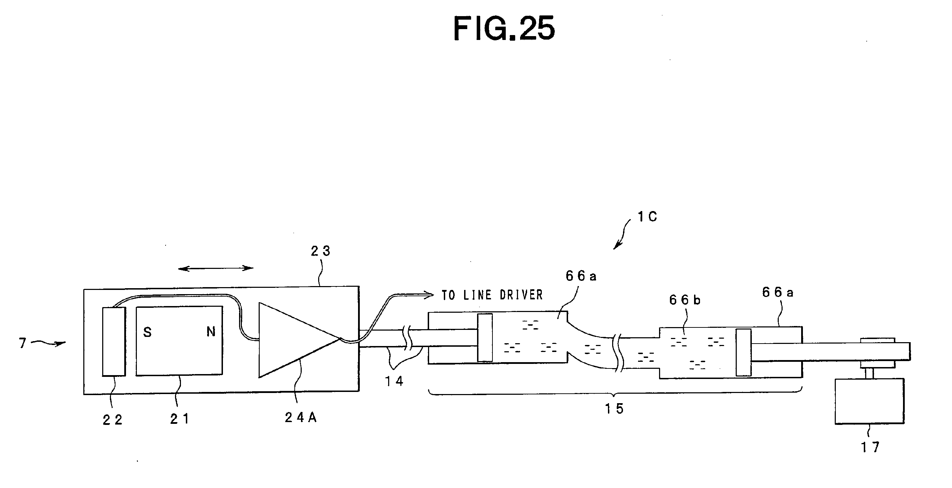

In a magnetic fluid detection device 1C shown in Fig. 25, the connector 15 has a hydraulic driving mechanism 66. The motor 17 is positioned far from the probe side using the hydraulic driving mechanism 66.

In the hydraulic driving mechanism 66, cylinders 60a are arranged on the probe side and on the motor side. The rotational movement of the motor 17 is converted to the advancing and receding motion, in which oil 66b is advanced and receded. Thus, the vibration is transmitted.

Thus, in the magnetic fluid detection device 1C, the vibration is transmitted to the vibration rod 14 by means of the hydraulic driving mechanism 66 of the connector 15.

Accordingly, in the magnetic fluid detection device 1C, the probe side is positioned far from the motor 17, so that the detection unit 7 is prevented from being affected by the motor magnet 60.

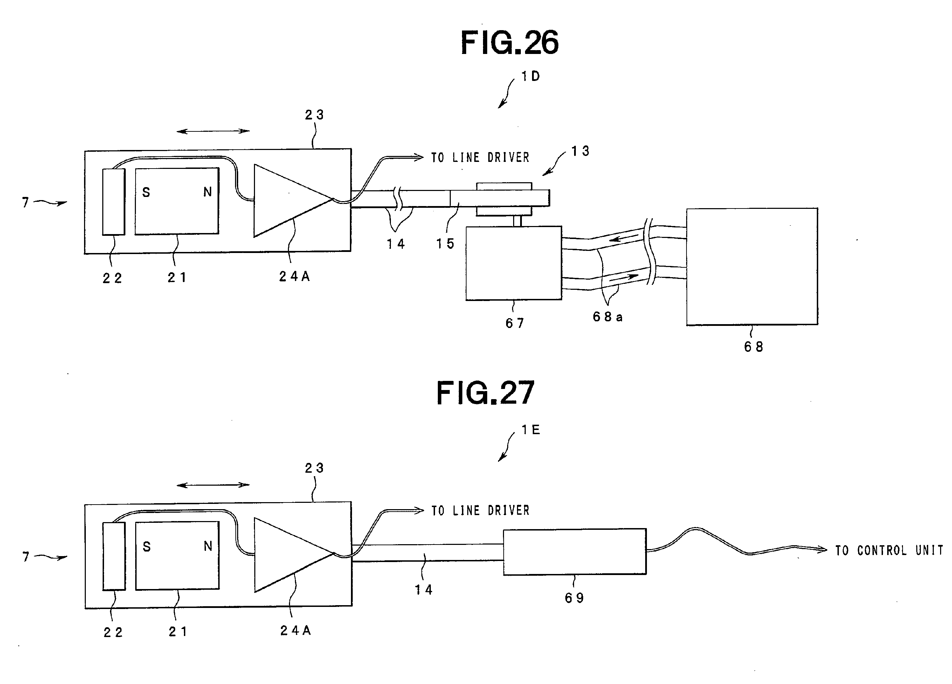

Moreover, in a magnetic fluid detection device 1D shown in Fig. 26, an air motor 67 is provided on the probe side. An air compressor 68 for driving the air motor 67 is positioned far from the probe side.

In the magnetic fluid detection device 1D, air is supplied to and discharged from the air compressor 68 via air tubes 68a so that the air motor 67 is rotated. The rotational motion is converted to the advancing and receding motion. Thus, the vibration is transmitted to the connector 15.

Thus, in the magnetic fluid detection device 1D, the air motor 67 with no magnets is provided on the probe side, and hence, the detection unit 7 is prevented from being affected by the motor magnet 60.

Furthermore, a magnetic fluid detection device 1E shown in Fig. 27 contains a supersonic motor or electrostatic actuator 69 provided with no magnets.

And, supplying driving current from the control unit 4, the magnetic fluid detection device 1E drives and controls the supersonic motor or electrostatic actuator 69 to transmit vibration to the vibration rod 14.

The magnetic fluid detection device 1E contains the supersonic motor or electrostatic actuator 69 provided with no magnets. Thus, the detection unit 7 is prevented from being affected by the motor magnet 60.

Second Embodiment

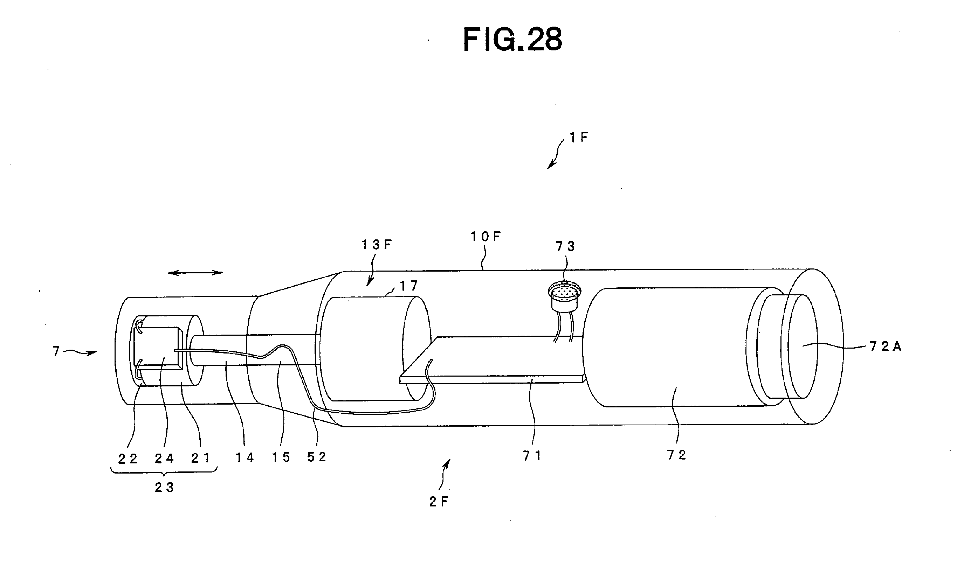

Figs. 28 to 38 show a magnetic fluid detection device according to a second embodiment of the present invention.

According to the first embodiment, the probe 2 and the control unit 4 are formed as separate pieces. According to the second embodiment, a control unit is contained in a probe. The other configuration is the same as that of the first embodiment. Thus, the description is not repeated. In the second embodiment, the same components as those of the first embodiment are designated by the same reference numerals.

That is, in the magnetic fluid detection device 1F according to the second embodiment, a probe 2F contains the control unit 4 as shown in Fig. 28.

The probe 2F is provided with a control substrate 71 having a control circuit mounted thereon as a control unit. The control substrate 71 is provided on the back side of the driving unit 13F. In the probe 2F, a battery 72 for supplying an electric power is provided on the back side of the control substrate 71. The control substrate 71 is provided with LED 73 as a display. The LED 72 is connected to the control substrate 71. The battery 72 may be charged with electromotive power from a charging coil 72A.

The sheath 10F of the probe 2F is formed so as to be transparent. Thus, the light-emitting state of the LED 73 can be seen through the probe sheath 10F. The driving unit 13F contains the motor 17.

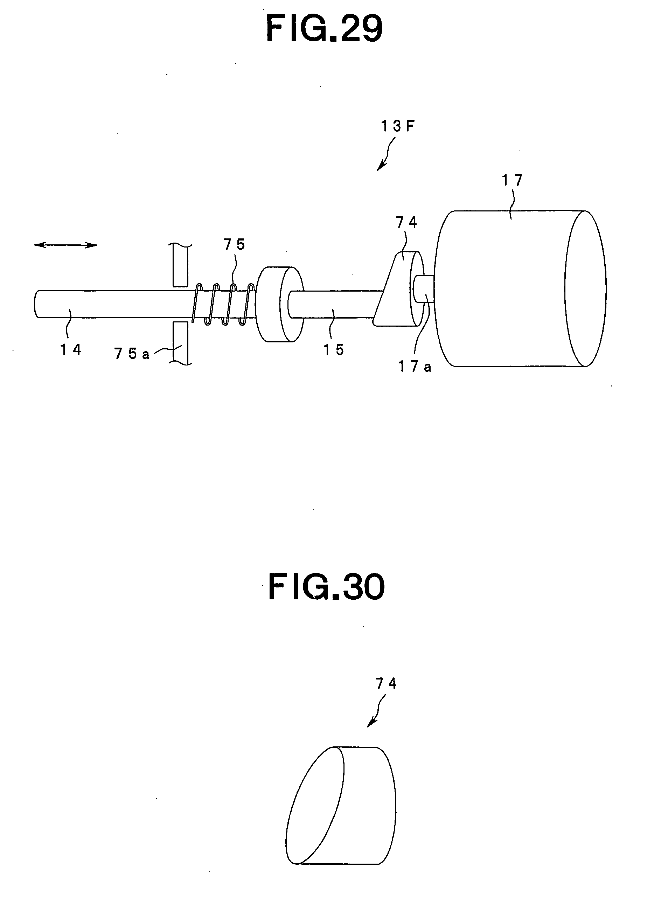

Specifically, as shown in Figs. 29 and 30, the driving unit 13F contains the motor 17 and an eccentric cam 74 disposed on an output shaft 17a of the motor 17.

The connector 15 is installed consecutively with the eccentric cam 74. The vibration rod 14 is biased with a spring 75 so as to be connected to the distal-end side of the connector 15. The spring 75, when it is pressed against the distal-end side of the connector 15, is given a biasing force by means of a spring-stopper 75a.

In the probe 2F, the motor 17 of the driving unit 13 is rotated against the biasing force of the spring 75 under control by the control circuit on the control substrate 71. Then, in the probe 2F, the rotational motion of the motor 17 is converted to the advancing and receding motion by means of the eccentric cam 74, and is transmitted to the connector 15. In the probe 2F, the vibration rod 14 is driven in the longitudinal axial direction through the connector 15.

Thus, the magnetic fluid detection device 1F has the advantages as those of the first embodiment.

Moreover, the size can be reduced, and its manipulation property is superior.

The coil 22 detects a change in magnetic flux (a change in magnetic flux density) passing through the aperture 22a. According to the Faraday's electromagnetic induction law, the output (electromotive voltage) signal from the coil 22 increases with the crossing speed rate over the magnetic flux being increased.

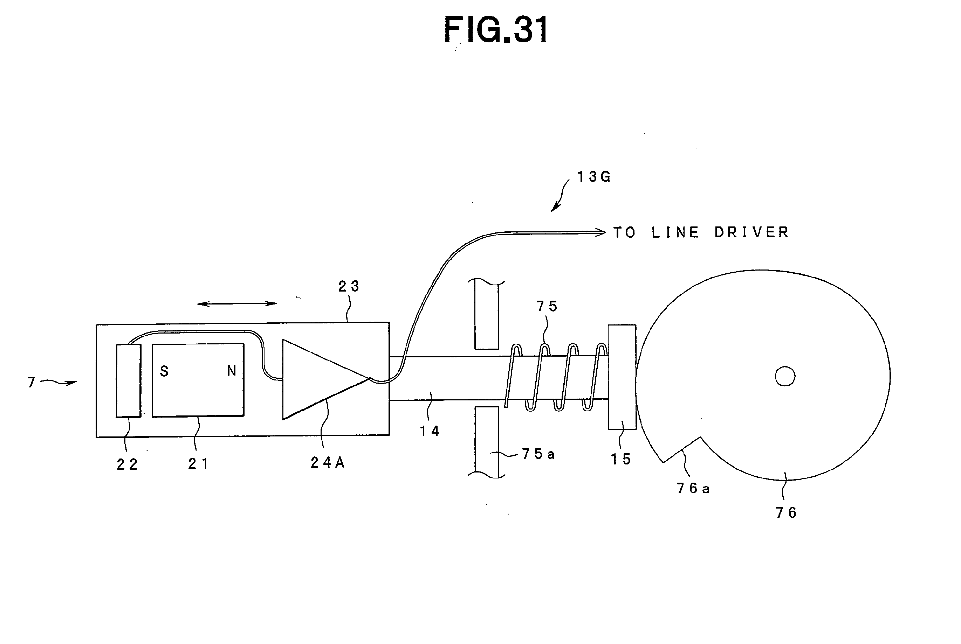

Thus, as shown in Figs. 31 to 34, the probe is configured so that the detection rate increases.

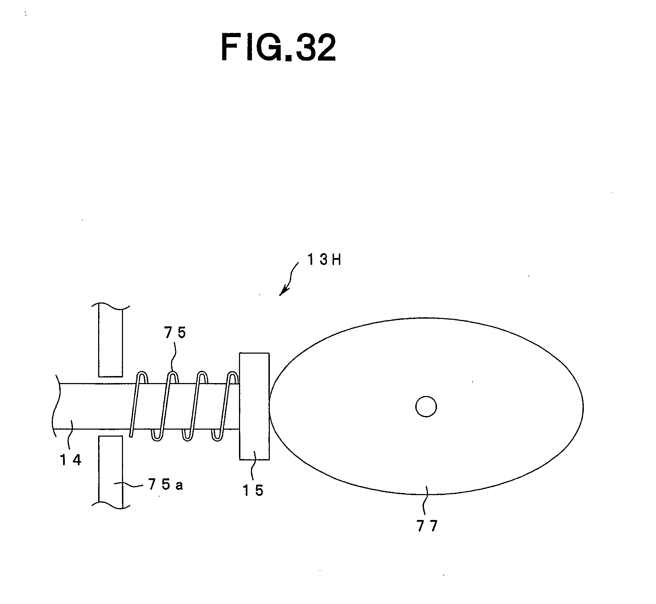

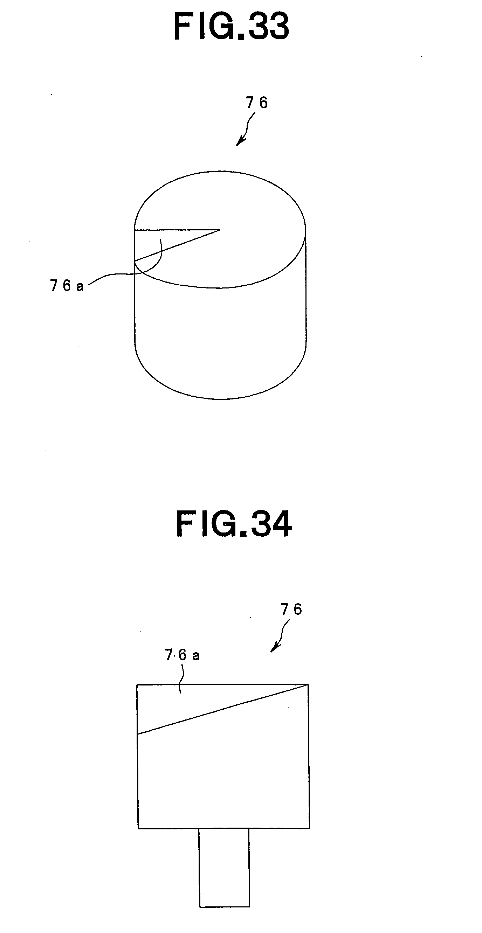

As shown in Fig. 31, a driving unit 13G contains a cam 76 having a step portion instead of the eccentric cam 74. As shown in Fig. 32, a driving unit 13H contains a elliptic cam 77 instead of the cam 76 having a step portion. The cam 76 has a step portion formed on the outer peripheral surface thereof. However, the step portion may be formed on an end-face of the cam 76, as shown in Figs. 33 and 34.

The connector 15 is rapidly (instantaneously) receded by using the cam 76 having a step portion of the driving unit 13G or by using the elliptic cam 77 of the driving unit 13H, so that the vibration rod 14 is rapidly receded. Thus, the coil 22 can output a very large output (electromotive voltage) signal.

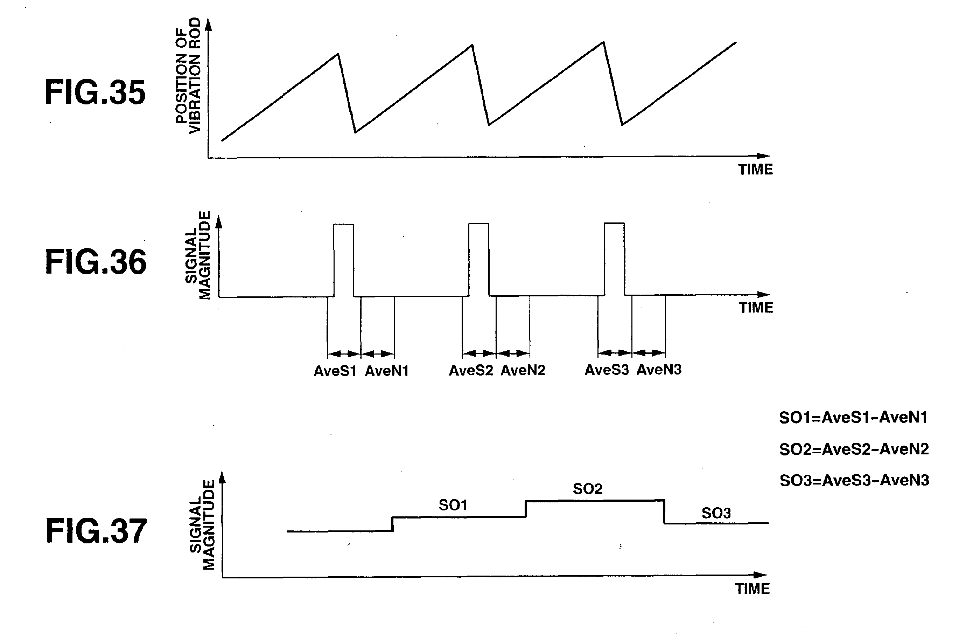

At this time, the vibration rod 14 is advanced and receded as shown in Fig. 35. The output signal of the coil 22 is generated as shown in Fig. 36, accompanying the advancing and receding motion.

The control circuit provided on the control substrate 71 carries out the following signal-processing of a signal output from the coil 22.

That is, the control circuit calculates the average values A ve S1, A ve S2, A ve S3, ⋯ of the magnitudes of the signal obtained when the vibration rod 14 is rapidly receded, and also, calculates the average values A ve N1, A ve N2, A ve N3, ... of the magnitudes of the signal obtained immediately after the receding.

The magnetic fluid 6 is detected in the time intervals in which the average values A ve S1, A ve S2, A ve S3, ⋯ of the magnitudes of the signal obtained when the vibration rod 14 is rapidly (instantaneously) receded are shown. Moreover, the speed of the vibration rod 14 is higher in these time intervals.

On the other hand, the vibration rod 14 is positioned farthest from the magnetic fluid 6 in the time intervals in which the average values A ve N1, A ve N2, A ve N3,⋯ is immediately after the receding. The magnitudes of the signal are not affected by the magnetic fluid 6, and also, the speed of the vibration rod 14 is low in these time intervals. That is, these signals represent noise components.

Therefore, these noise components can be eliminated by subtracting the average values A ve N1, A ve N2, A ve N3,⋯ , obtained immediately after the vibration rod 14 is receded, from the average values A ve S1, A ve S2, A ve S3 ,⋯ , obtained when the vibration rod 14 is rapidly (instantaneously) receded.

Moreover, the measuring time interval in which the magnitude of the signal is averaged, is set so as to be equal to integer times the one period of a commercial electric source. Thus, the noise of the commercial electric source is averaged based on the one period or integer times the one period. Thus, the value of the noise becomes substantially zero. Therefore, the effect of the noise of the commercial electric source can be substantially eliminated.

Then, the above-described signal processing is carried out. Fig. 37 shows the resultant signal.

In Fig. 37, SO1 is a value obtained by subtracting A ve N1 from A ve S1, SO2 is a value obtained by subtracting A ve N2 from A ve S2, and SO3 is a value obtained by subtracting A ve N3 from A ve S3.

The control circuits, after SO1 is obtained, keeps SO1 until the next SO2 is obtained. After SO2 is obtained, the control circuit keeps SO2 until the next SO3 is obtained. Thereby, a signal showing whether the magnetic fluid 6 is present or absent is obtained. Thus, the magnetic fluid detection device F can detect the magnetic fluid 6 more accurately.

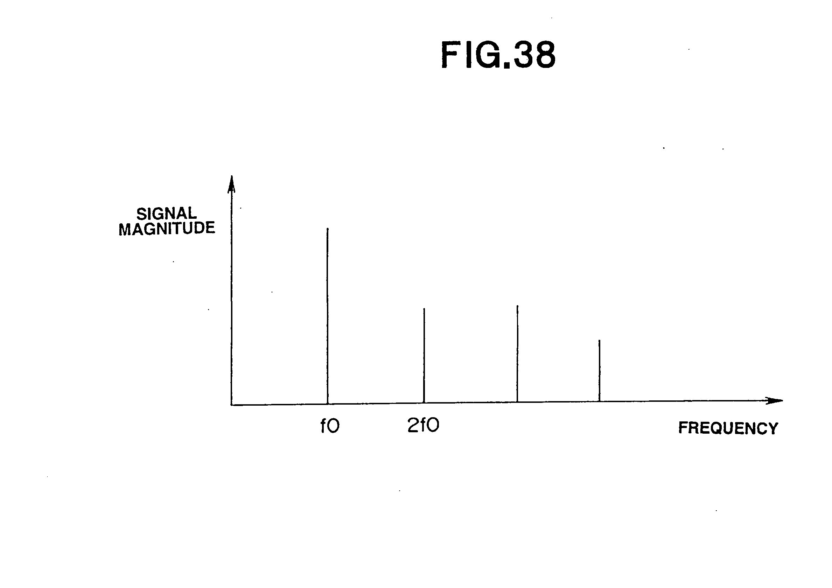

Moreover, in the control circuit, e. g., a higher harmonic component 2f0 with respect to the fundamental frequency f0, which is the vibration frequency, is generated as shown in Fig. 38. Thereby, the rotation noise of the motor 17 can be removed from the fundamental frequency f0, on which the rotation noise of the motor 17 is easily superposed.

Third Embodiment

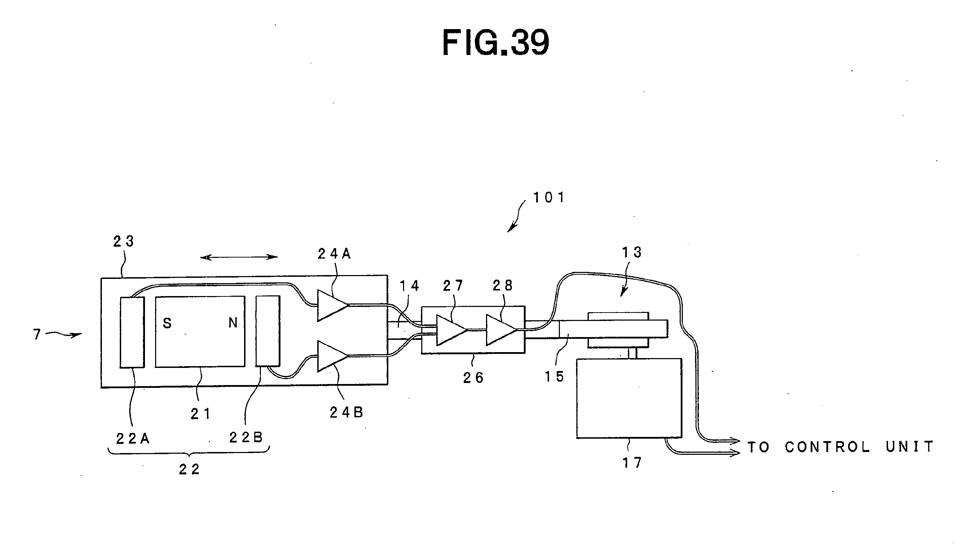

Figs. 39 to 46 show a magnetic fluid detection device according to a third embodiment of the present invention.

According to the first and second embodiments, one coil 22 is used as a magnetic sensor. In the third embodiment, plural magnetic sensors are used. Other configuration is the same as that of the first embodiment, and the description is not repeated. The same components as those of the first embodiment are designated by the same reference numerals.

In particular, as shown in Fig. 39, a magnetic fluid detection device 101 contains plural coils 22 (two coils 22A and 22B) as magnetic sensors.

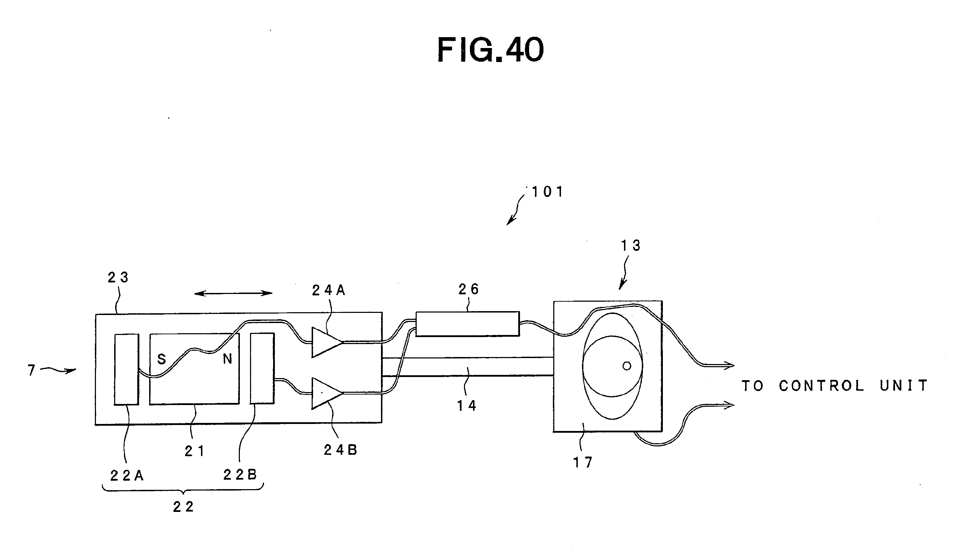

The coil 22A is disposed on the distal-end side of the detection unit body 23, and is exposed on the distal-end portion of the vibration rod 14. The exciting magnet 21 is arranged on the back side of the coil 22A. Moreover, the coil 22B is arranged on the back side of the exciting magnet 21. That is, the exciting magnet 21 is arranged between the coil 22A and coil 22B. As shown in Fig. 40, in the detection unit 7, the vibration rod 14 may be connected directly to the motor 17 of the driving unit 13.

According to this embodiment, the output of one of the plural coils 22A and 22B is subtracted from that of the other coil in order to eliminate magnetic noise, e.g., caused by the terrestrial magnetism, the effect of the magnetic field generated by the motor magnet of the motor 17, or the like.

Moreover, according to this embodiment, the difference between the outputs of the plural coils 22 (coil 22A and coil 22B) is calculated, the magnetic noise caused by the terrestrial magnetism or the like is removed, and then, the vibration frequency component is detected, as described below. Thus, in this manner, the magnetic noise caused by the terrestrial magnetism, electrical devices or apparatuses, and so forth, is removed.

The apertures of the plural coils 22 are set at not more than 1 cm, i.e., are set so as to be smaller than a lymph node, as described in the first embodiment. Thereby, the range where the coils 22 (22A and 22B) detect magnetic noise occurring due to electrical devices or apparatuses can be minimized, and thus, the coils 22 can detect only the magnetic force lines generated by the magnetic fluid 6.

In the pre-amplification portion 24, plural pre-amplification portions, that is, pre-amplification portions 24A and 24B for amplifying the outputs from the plural coils 22 (coils 22A and 22B) are provided. The pre-amplification portion 24A amplifies the output from the coil 22A, and the pre-amplification portion 24B amplifies the output from the coil 22B.

The line driver 26 is provided with a subtracter 27 for calculating the difference between the outputs of the plural coils 22 (22A and 22B), and an amplifier 28 for amplifying the output from the subtracter 27.

The output from the line driver 26 is transmitted to the control unit 4, and is signal-processed therein.

The configuration of the control unit 4 is the same as that descried in the first embodiment, and the description is not repeated.

As described above, according to this embodiment, the plural coils 22, e.g., the coils 22A and 22B are used.

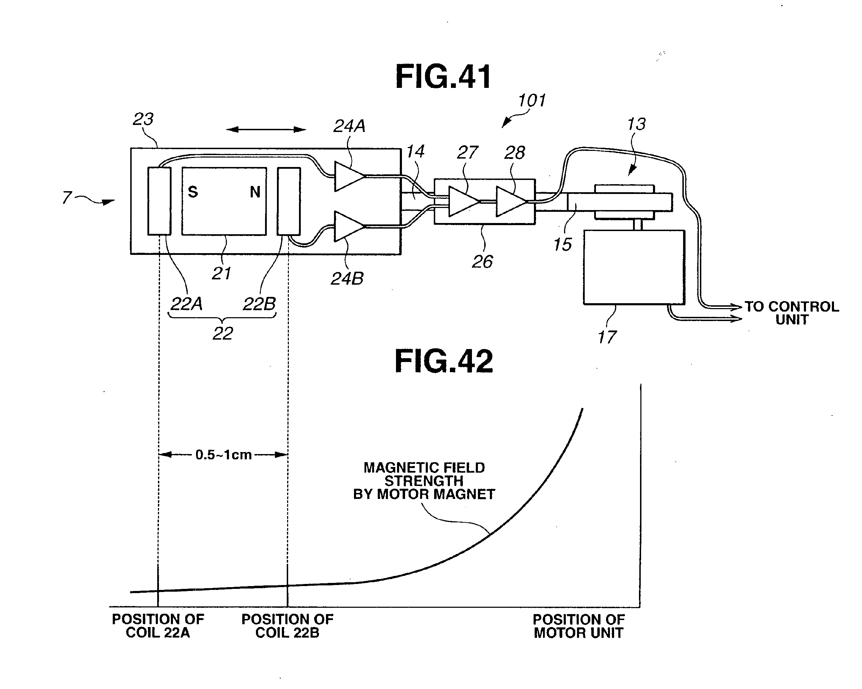

As shown in Figs. 41 and 42, the magnetic field generated by a motor magnet (not shown) of the motor 17 is applied to the coils 22. The strengths of the magnetic field applied to the coils 22 change with the distances between the coils 22 and the motor magnet. When the coils 22 are vibrated, the strengths of the magnetic field applied to the coils 22 change. Thus, according to the Faraday's electromagnetic induction law, voltages are output from the coils 22. The voltages have no relation to the magnetic fluid, and hence, become noise when the magnetic fluid is detected.

The magnetic field from the motor magnet is exponentially attenuated proportionally to the distance from the motor magnet in the vicinity of the motor magnet. However, the gradient of the electromagnetic field attenuation may be estimated to be constant in the range of a few centimeters in the vicinity of the plural coils 22 which are significantly distant from the motor magnet.

Accordingly, it may be estimated that when the detection unit 7 is vibrated, the magnitudes of the electromagnetic field applied from the motor magnet to the coils 22A and 22B change to the same degrees, and thus, the voltages output from the coils 22A and 22B are equal.

Accordingly, the effects of the motor magnets on the coils 22A and 22B can be eliminated by subtracting the output of one of the coils 22A and 22B from that of the other coil.

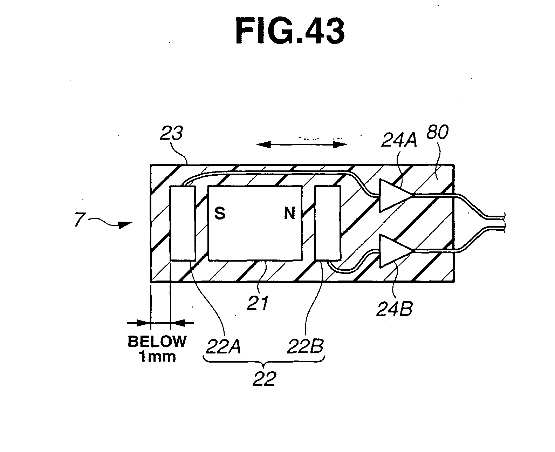

As shown in Fig. 43, a resin 80 is filled into the spaces existing in the body 23 of the detection unit 7 and hardened so that the plural coils 22 (22A and 22B), the exciting magnet 21, and the pre-amplification portion 24 contained in the detection unit body 23 are fixed. That is, the coils 22A and 22B are arranged in parallel and fixed by means of the resin 80. Therefore, when the detection unit 7 is vibrated accompanying the vibration of the vibration rod 14, the directions and the positions of the coils 22A and 22B are prevented from relatively changing.

According to this embodiment, the detection unit 7 is vibrated, e.g., over a length of 1 to 2 mm in the longitudinal axial direction accompanying the vibration of the vibration rod 14 in the longitudinal axial direction. Therefore, for the vibration of the detection unit 7, a space having a size of 1 to 2 mm is provided between the detection unit 7 and the probe sheath 10.

In general, the magnitude of a magnetic field decreases inversely proportional to the square of the distance from a vibration source. Therefore, desirably, the coil 22A is located as near to the distal-end side of the detection unit 7 as possible.

Thus, according to this embodiment, the coil 22A is located at a position of not more than 1 mm from the distal end of the detection unit body 23.

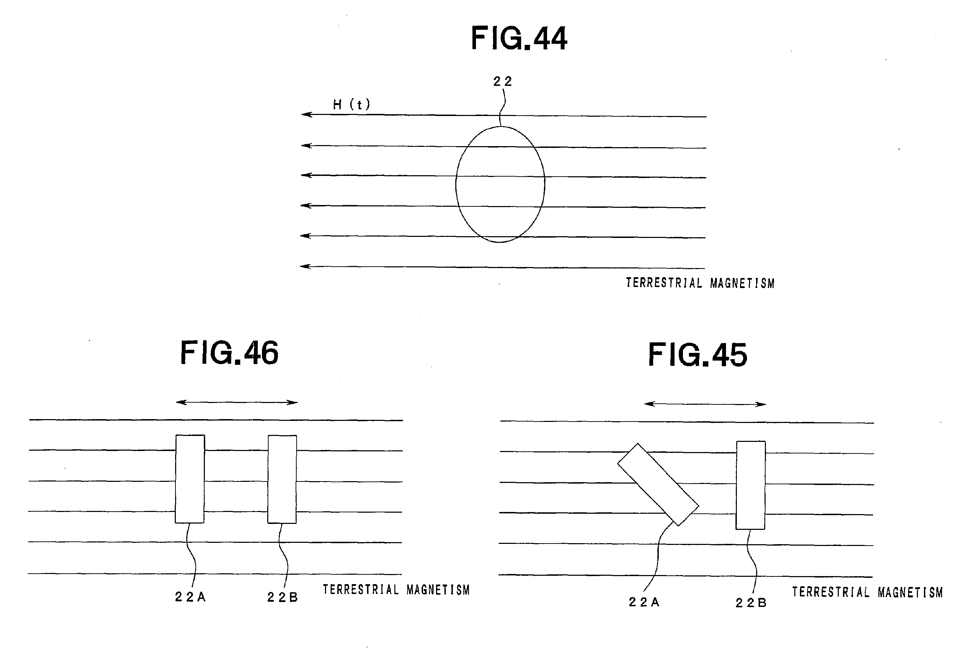

It is assumed that the magnetic force lines of the terrestrial magnetism extend perpendicularly across the coil 22, as shown in Fig. 44.

As shown in Fig. 45, the coils 22A and 22B are relatively moved, due to the vibration of the detection unit 7. Thus, their relative directions and positions are changed. Then, the magnetic force lines of the terrestrial magnetism extending across the coils 22A and 22B change. Accordingly, even if the subtraction is carried out on the outputs of the coils 22A and 22B, the magnetic noise, generated by the resultant magnetic field formed of the magnetic field by the motor magnet and the terrestrial magnetism as described above, cannot be eliminated.

However, according to this embodiment, the directions and the positions of the coils 22A and 22B are prevented from relatively changing, although the detection unit 7 is vibrated accompanying the vibration of the vibration rod 14. Therefore, the magnetic force lines of the terrestrial magnetism extending across the coils 22A and 22B change in the same manners with respect to the coils 22A and 22B.

Therefore, according to this embodiment, the magnetic noise, which is generated by the resultant magnetic field formed from the magnetic field by the motor magnet and that by the terrestrial magnetism, as described above, can be eliminated by the subtraction of the outputs of the coils 22A and 22B.

The magnetic fluid detection device 101 is applied to detect the magnetic fluid 6 staying in the sentinel lymph node 5 of a subject to identify the sentinel lymph node 5.

First, an operator punctures the lower layer of a lesion of the subject with a puncture needle (not shown), and infuses the magnetic fluid 6 locally in the vicinity of the lesion. Then, the magnetic fluid 6 infused in the vicinity of the lesion is moved from the infusion position to a lymph vessel, reaches the sentinel lymph node from five to fifteen minutes after the infusion, and stays in the sentinel lymph node 5.

Then, the operator surgically inserts the probe 2 of the magnetic fluid detection device 101 into an intracavity, e.g., via a trocar (not shown), or is placed on the surface of the subject body from the outside of the body. The operator detects the magnetic fluid 6 staying in the sentinel lymph node 5 while the operator moves the distal end of the probe 2 in the vicinity of the lesion of the patient.

Then, in the probe 2, the motor 17 of the driving unit 13 is driven while it is controlled by the motor control circuit 36 of the control unit 4. The rotational motion of the motor 17 is converted to the advancing and receding motion, and the vibration is transmitted to the connector 15.

In the probe 2, the vibration rod 14 is vibrated in the longitudinal axial direction by the vibration transmitted from the driving unit 13 via the connector 15, while the vibration rod 14 is slid and guided by the guides 16a and 16b. Thereby, the detection unit 7 is vibrated in the longitudinal axial direction. In the probe 2, the exciting magnet 21 of the detection unit 7 is vibrated in the longitudinal axial direction. Thus, the probe 2 generates an AC magnetic field depending on the vibration frequency.

When the magnetic fluid 6 exists in the vicinity of the lesion of the patient, the AC magnetic field generated by the exciting magnet 21 excites the magnetic fluid 6 via the space in the vicinity of the probe. Then, the AC magnetic field is attracted or repelled in the vicinity of the magnetic fluid 6, so that the magnetic field distribution is locally distorted, and thus, the spatial gradient (magnetic flux density) of the magnetic field distribution changes. This local distortion of the magnetic field distribution (the change of the magnetic flux density), occurring due to the magnetic fluid 6, is detected by the plural coils 22 (22A and 22B).

In this case, the coils 22A and 22B can detect the local distortion of the magnetic field distribution (the spatial magnetic gradient) occurring due to the magnetic fluid 6 without being influenced with the exciting magnetic field (the AC magnetic field). Outputs from the coils 22A and 22B are amplified by the pre-amplifiers 24A and 24B, and are transmitted to the line driver 26.

In the line driver 26, the subtracter 27 carries out the subtraction of the outputs from the coils 22A and 22B, and the difference is amplified by the amplifier 28 and transmitted to the control unit 4.

In this case, in the detection unit 7, the pre-amplification portion 24, together with the exciting magnet 21 and the plural coils 22 (22A and 22B), is vibrated in the longitudinal axial direction, accompanying the vibration of the vibration rod 14 in the longitudinal axial direction. Thus, lead wires between the coils 22 (22A and 22B) and the pre-amplification portion 24 are not vibrated, so that no change in the contact resistance or the like occurs, and hence, the control unit 4 is not affected by such change.

The lead wires between the pre-amplification portion 24 and the line driver 26 is vibrated. However, the fine outputs from the coils 22 are amplified in the pre-amplification portion 24. Thus, even if the signal is varied by a change in contact resistance or the like, the change of the signal is slight compared to the signal magnitude after the amplification is carried out. Thus, such change of the contact resistance or the like does not exert an influence on the output signal substantially.

Thus, noise can be prevented from being generated due to the vibration of the coils 22 (22A and 22B) and the exciting magnet 21.

In the control unit 4, the higher harmonic component of the output signal received by the line receiver 31 is eliminated therefrom by LPF 32, and the amplitude component is taken out. The amplitude component taken out is amplified by the amplifier 33, and is A/D converted by the A/D converter 34.

In the control unit 4, the digital signal processing circuit 35 carries out digital-signal-processing such as high speed Fourier transformation or the like of the outputs from the plural coils 22 (22A and 22B) (the digital signal from the A/D converter 34), based on the pulse signal from the motor control circuit 36, thereby to detect the amplitude of the vibration frequency component. Thus, the display 8 and the speaker 9 are driven in response to the detected signal magnitude.

The display 8 and the speaker 9 operate in the same manners as described in the first embodiment to inform the operator.

Thus, the magnetic fluid detection device 101 of the third embodiment can accurately detect the position of the magnetic fluid 6 staying in the sentinel lymph node 5 to identify the position of the sentinel lymph node 5 without being affected by the magnetic noise of the terrestrial magnetism or the like.

Fourth Embodiment

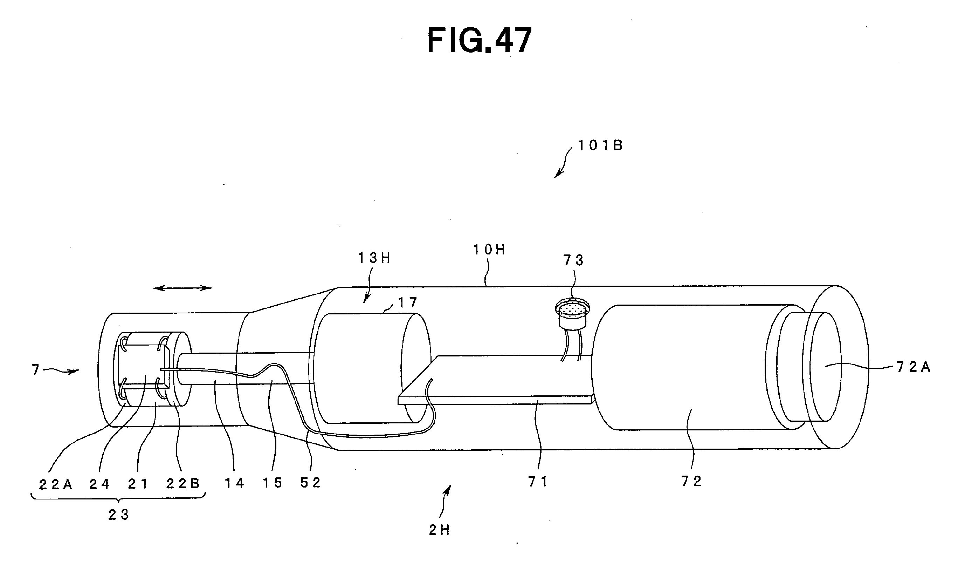

Figs. 47 to 54 show a magnetic fluid detection device according to a forth embodiment of the present invention.

According to the third embodiment, the probe 2 and the control unit 4 are formed as separate pieces. According to the fourth embodiment, a control unit is contained in a probe. The other configuration is the same as that of the third embodiment. Thus, the description is not repeated. In the fourth embodiment, the same components as those of the third embodiment are designated by the same reference numerals.

That is, in the magnetic fluid detection device 101B according to the fourth embodiment, a probe 2H contains the control unit 4 as shown in Fig. 47.

The probe 2H is provided with the control substrate 71 having a control circuit mounted thereon as a control unit. The control substrate 71 is provided on the back side of the driving unit 13H. In the probe 2H, the battery 72 for supplying an electric power is provided on the back side of the control substrate 71. The control substrate 71 is provided with LED 73 as a display. The LED 72 is connected to the control substrate 71. The battery 72 may be charged with electromotive power from the charging coil 72A.

The sheath 10H of the probe 2H is formed so as to be transparent. Thus, the light-emitting state of the LED 73 can be seen through the probe sheath 10H. The driving unit 13H contains the motor 17.

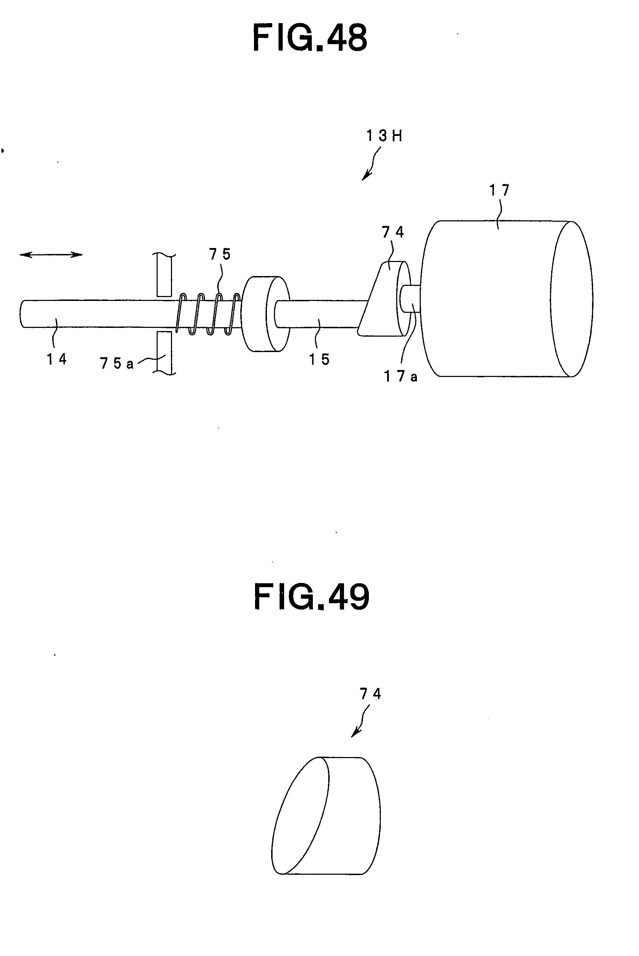

Specifically, as shown in Figs. 48 and 49, the driving unit 13H contains the motor 17 and the eccentric cam 74 disposed on the output shaft 17a of the motor 17.

The connector 15 is installed consecutively with the eccentric cam 74. The vibration rod 14 is biased with a spring 75 so as to be connected to the distal-end side of the connector 15. The spring 75, when it is pressed against the distal-end side of the connector 15, is given a biasing force by means of the spring-stopper 75a.

In the probe 2H, the motor 17 of the driving unit 13 is rotated against the biasing force of the spring 75 under control by the control circuit on the control substrate 71. Then, in the probe 2H, the rotational motion of the motor 17 is converted to the advancing and receding motion by means of the eccentric cam 74, and is transmitted to the connector 15. In the probe 2H, the vibration rod 14 is vibrated in the longitudinal axial direction through the connector 15.

Thus, the magnetic fluid detection device 1H of the fourth embodiment has the advantages as those of the first embodiment. Moreover, the size can be reduced, and its manipulation property is superior, since the probe contains the control unit.

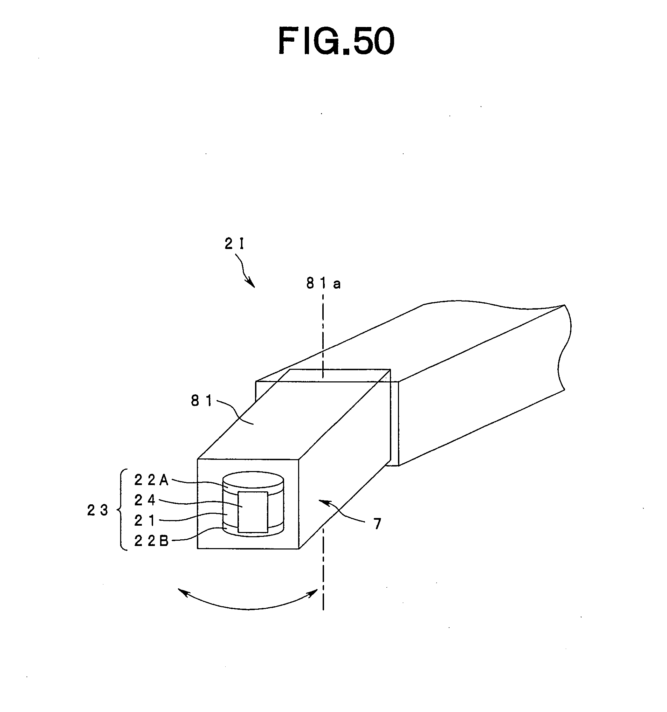

The probe may be configured in such a manner that the distal-end portion of the probe can swing (be shaken) in the right and left direction, and thereby the probe is vibrated in the right and left direction, as shown in Fig. 50.

As shown in Fig. 50, the probe 2I is configured in such a manner that the distal-end portion 81 thereof can swing in the right and left direction about a fulcrum 81a as a center. Therefore, the movement amount of the detection unit 7 in the right and left direction increases. Thus, the movement speed of the probe 2I can be increased.

In particular, the coils 22 (22A and 22B) detect a change in the magnetic flux extending across the aperture (a change in the magnetic flux density). Thus, as the change of the magnetic flux per unit time increases, the outputs (electromotive voltage) signal from the coils 22 increases according to the Faraday's electromagnetic induction law.

Thus, in the probe 2I, the movement speed of the detection unit 7 is increased, and thus, the movement speeds of the coils 22A and 22B are increased, so that the coils 22A and 22B output large output (electromotive voltage) signals.

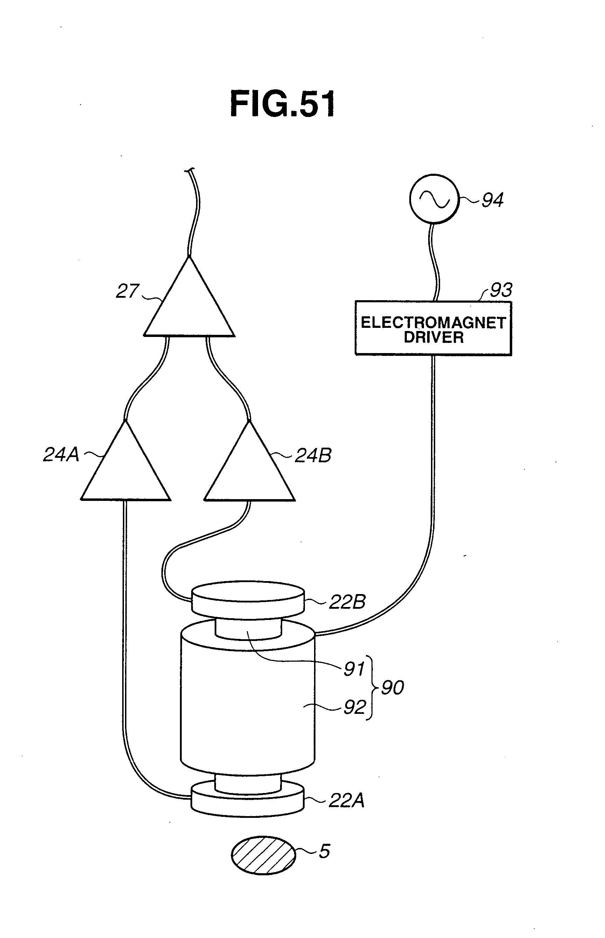

In the third and fourth embodiments, a permanent magnet is used as the exciting unit comprising the exciting magnet 21. The exciting magnetic field generated by the exciting magnet 21 is an AC magnetic field. The exciting unit may comprise an exciting electromagnet 90 shown in Fig. 51.

As shown in Fig. 51, the exciting electromagnet 90 is formed by winding a coil 92 around an iron core 91. AC current is supplied from an AC source 94 to the coil 92 of the exciting electromagnet 90 via an electromagnet driver 93. The electromagnet driver 93 is driven under control by the control unit 4.

The magnetic fluid 6 staying in the subject is excited with the AC magnetic field generated by the exciting electromagnet 90. The local distortion of the magnetic field distribution (spatial magnetic gradient), generated by the magnetic fluid 6, is detected by the coils 22 (22A and 22B).

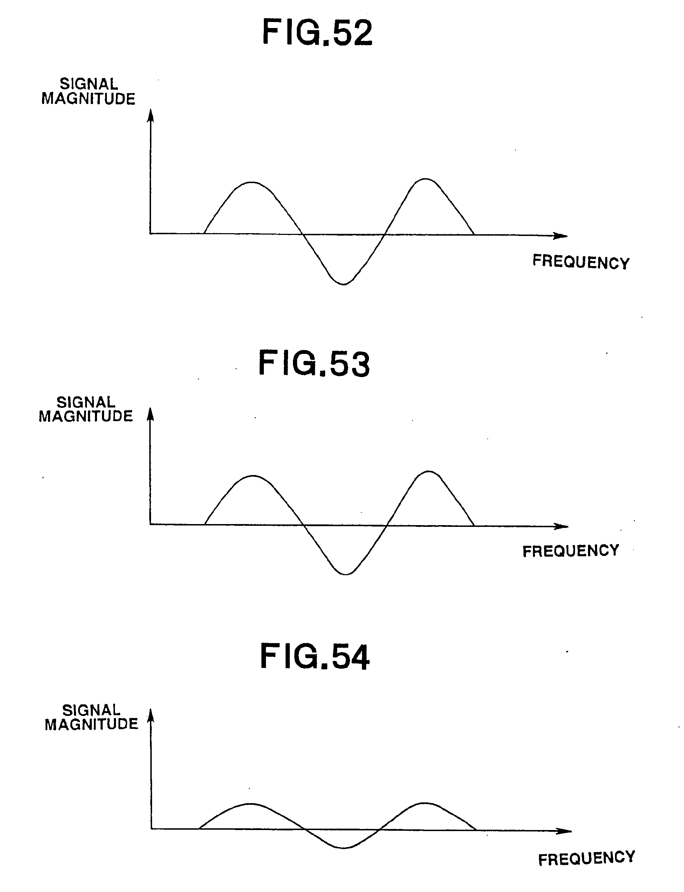

The outputs of the coils 22 (22A and 22B) are shown in Figs. 52 and 53. The subtraction between the outputs of the coils 22 (22A and 22B) is carried out by the subtracter 27. The difference signal is shown, e.g., in Fig. 54.

If no magnetic fluid 6 exists, the output signals from the coils 22A and 22B have an equal magnitude. The difference in magnitude between the magnitudes of the output signals becomes zero, e.g., in Fig. 54.

If the magnetic fluid 6 exists, the output signal from the coil 22A, positioned relatively near to the magnetic fluid 6, is higher than that from the coil 22B. The signal magnitude obtained by subtracting one from the other is exhibited depending on the frequency of the AC magnetic field, as shown in Fig. 54.

Thus, the magnetic fluid detection device using the exciting electromagnet 90 instead of the exciting magnet 21 can detect the magnetic fluid 6.

Thus, it becomes unnecessary to provide a vibration mechanism for the magnetic fluid detection device. The structure of the magnetic fluid detection device is simple. The size can be reduced, and the manipulation property is superior.

Fifth Embodiment

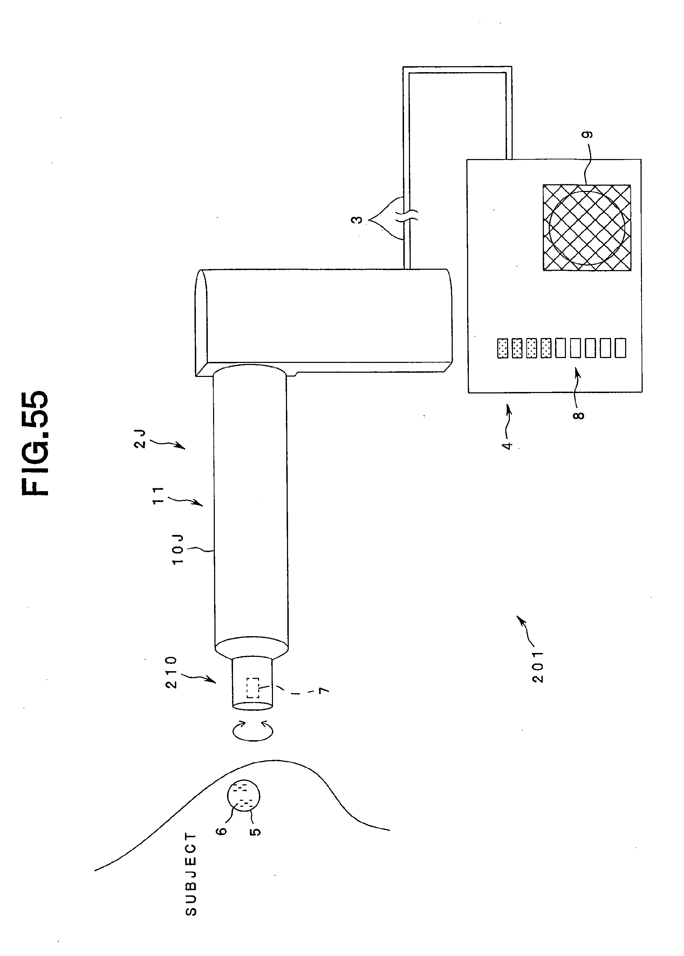

Figs. 55 to 58 show a magnetic fluid detection device according to a fifth embodiment of the present invention.

According to the third embodiment, the detection unit 7 is vibrated in the longitudinal axial direction. According to the fourth embodiment, the detection unit 7 is caused to swing in the right and left direction. On the other hand, according to the fifth embodiment, the detection unit 7 can be revolved in an optional direction, whichever it may be a clockwise or counterclockwise direction. The other configuration is the same as that of the third embodiment, and the description is not repeated. The same components are designated by the same reference numerals.

As shown in Fig. 55, a magnetic fluid detection device 201 of the fifth embodiment contains a probe 2J. The probe 2J is provided with a distal-end revolution portion 210 capable of being revolved with respect to the probe body 11.

The distal-end revolution portion 210 and the probe body 11 are covered with a probe sheath 10J made of a non-magnetic material. The distal-end revolution portion 210 is water-tight so that the probe 2J can be inserted into an intracavity for detection.

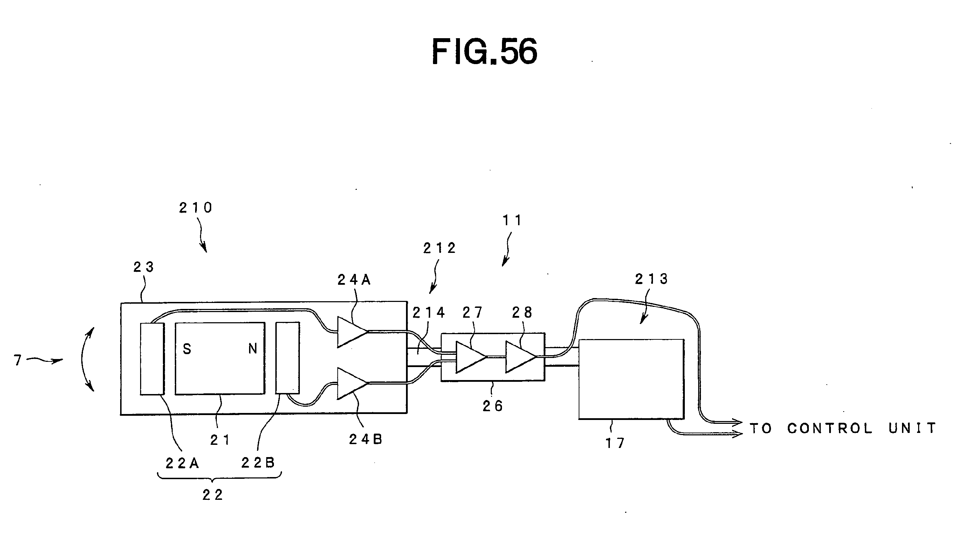

As shown in Fig. 56, the probe body 11 comprises a revolution unit 212 with which the distal-end revolution portion 210 is revolved, and a revolution-driving unit 213 for revolving the revolution unit 212.

The revolution unit 212 contains a revolution member 214 formed of a non-magnetic material and being capable of revolving around its longitudinal axis. The proximal end of the revolution member 214 is connected to the revolution-driving unit 213. The revolution motion from the revolution-driving unit 213 is transmitted to the distal-end revolution portion 210 via the revolution member 214.

The revolution-driving unit 213 transmits the rotation of the motor 17 to the revolution member 214 of the revolution unit 212. That is, the revolution-driving unit 213 and the revolution member 214 constitute a revolution portion.

The distal-end revolution portion 210 can be revolved around its longitudinal axis. The revolution-driving unit 213 may use a supersonic motor or an electrostatic actuator (not shown) instead of the motor 17, which causes the revolution member 214 to revolve around its longitudinal axis.

The detection unit 7 is provided in the distal-end portion of the distal-end revolution portion 210.

The body 23 of the detection unit 7 comprises the exciting magnet 21 and the coil 22. In the detection unit 7, the coil 22 comprises plural coils, e.g., coils 22A and 22B as in the third embodiment.

In the detection unit 7, the exciting magnet 21 can be revolved around its longitudinal axis accompanying the revolution of the distal-end revolution portion 210. Thus, in the detection unit 7, an AC magnetic field depending on the revolution frequency is generated as an exciting magnetic field, with which the magnetic fluid 6 staying in the subject is detected.

According to this embodiment, the plural coils 22 (22A and 22B) are used as in the third embodiment. Thus, magnetic noise generated by the effects of magnetic fields occurring due to the terrestrial magnetism, motor magnets of the motor 17, and so forth can be eliminated by subtraction of the outputs from the coils 22 (22A and 22B).

According to this embodiment, the size of the aperture of each of the coils 22 (22A and 22B) is set at 1 cm or smaller, that is, is smaller than that of a lymph node. Thus, the region of the coil 22 in which the coil 22 detects magnetic noise generated by electrical devices or apparatuses and so forth can be minimized, so that only the magnetic force lines from the magnetic fluid 6 can be detected.

Moreover, according to this embodiment, the magnetic noise generated by the terrestrial magnetism and so forth is eliminated by subtraction of the outputs from the coils 22 (22A and 22B), and thereafter, the revolution frequency component is detected, as described below.

The detection unit 7 contains the pre-amplification portion 24. In the pre-amplification portion 24, plural pre-amplifiers, that is, pre-amplifiers 24A and 24B for amplifying the outputs from the plural coils 22 (coils 22A and 22B) are provided. The preamplifier 24A amplifies the output from the coil 22A, and the preamplifier 24B amplifies the output from the coil 22B.

That is, in the detection unit 7, the pre-amplification portion 24, together with the exciting magnet 21 and the plural coils 22 (22A and 22B), can be revolved around the longitudinal axial direction of the vibration rod 14, integrally with the revolution of the vibration rod 14 around its longitudinal axial direction.

According to this embodiment, the lead wires between the coils 22 and the pre-amplification portion 24 are prevented from being relatively revolved. Thus, the effects of the change of the contact resistances can be eliminated.

The lead wires between the pre-amplification portion 24 and the line driver 26 are relatively revolved. However, the pre-amplification portion 24 and the line driver 26 are electrically connected to each other via slip rings as described below. Moreover, the very small outputs from the coils 22 are amplified by the preamplifiers 24A and 24B. Thus, even if the signals from the coils 22 are changed with the contact resistances or the like, the changing degree is very small compared to the magnitudes of the amplified signals, and thus, is negligible.

In the revolution unit 212, the line driver 26 for transmitting the outputs from the detection unit 7 to the control unit 4 is arranged and fixed in the vicinity of the revolution member 214, as a separate piece with respect to the revolution member 214. That is, the line driver 26 is prevented from revolving. Accordingly, the weight of the revolution member 214 is prevented from increasing, which would occur if the relatively heavy line driver 26 is attached to the revolution member 214.

The line driver 26 is provided with the subtracter 27 for subtracting the outputs from the plural coils 22 (22A and 22B) and an amplifier 28 for amplifying the output from the subtracter 27.

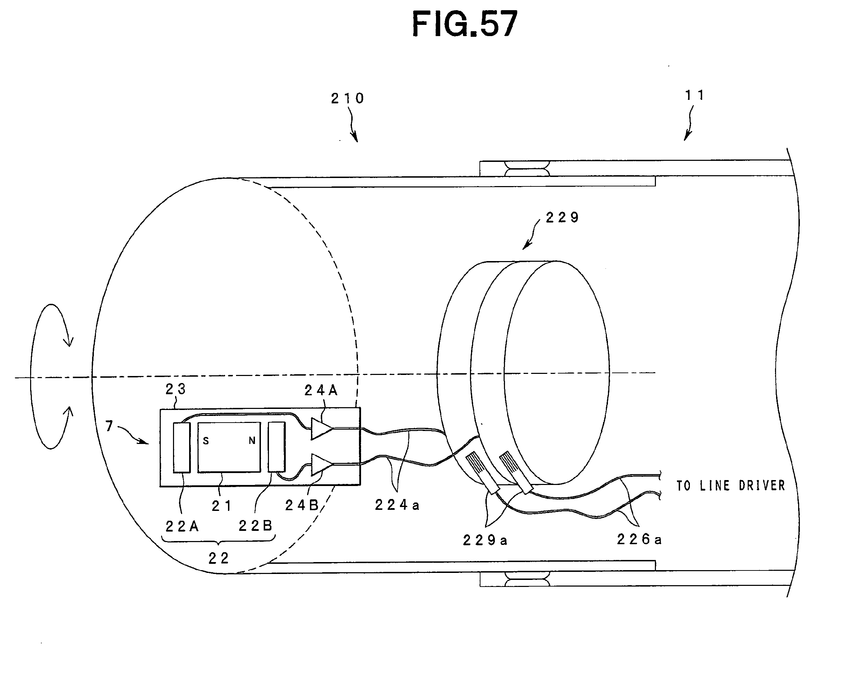

The distal-end revolution portion 210 is revolved with respect to the probe body 11 by means of the revolution member 214. For this purpose, as shown in Fig. 57, in the distal-end revolution portion 210, the detection unit 7 is electrically connected to the probe body 11 via a slip ring 229.

The distal-end side of the slip ring 229 is connected to lead wires 224a extended from the pre-amplification portion 24, and the rear-end side thereof is connected to electrode brushes 229a provided on the ends of lead wires 226a extended from the line driver 26. Thus, the pre-amplification portion 24 is electrically connected to the line driver 26. In the probe 2J, with the slip ring 229 used, the lead wires extended to the detection unit 7 can be prevented from twisting to be broken or disconnected, which will occur by the revolution transmitted from the revolution member 214.

In the above-described case, if the coils 22 (22A and 22B) are arranged so that the longitudinal axes of the coils 22 coincide with the center line of the revolution, the local distortion of the magnetic field distribution (spatial magnetic gradient), caused by the magnetic fluid 6, will not change, irrespective of the site of the magnetic fluid 6, although the coils 22 (22A and 22B) are revolved. Thus, the signal change can not be detected.

Therefore, according to this embodiment, the detection unit 7 is located so that the coils 22 can be positioned eccentrically with respect to the center of the revolution.

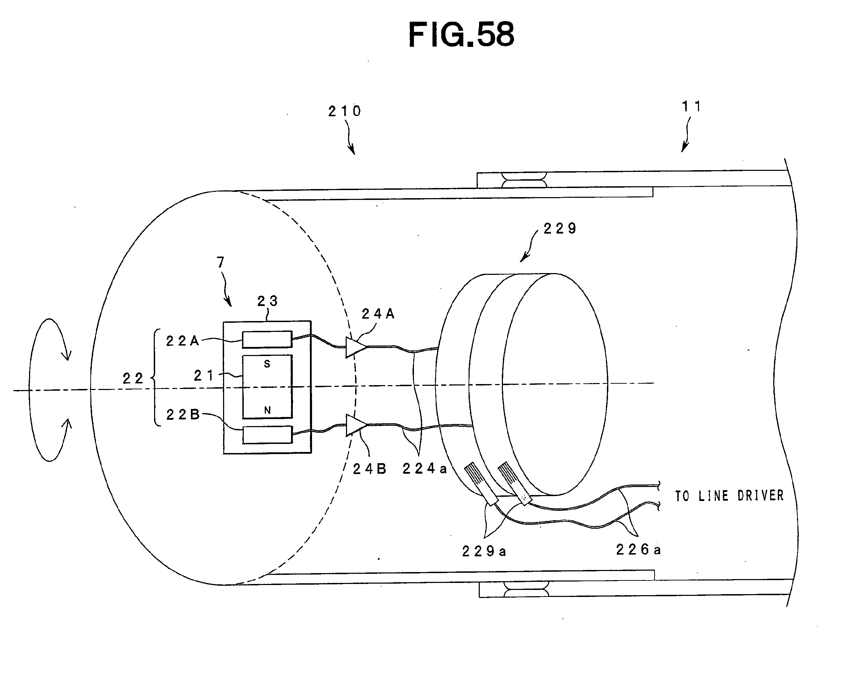

Moreover, as shown in Fig. 58, the detection unit 7 may be positioned so that the center line of the detection unit 7 coincides with that of the revolution, in which the coils 22 (22A and 22B) are located eccentrically with respect to the center line of the revolution. The output from the line driver 26 is transmitted to the control unit 4, which carries out the signal-processing.

The control unit 4 has almost the same configuration as that in the first embodiment except that the digital signal processing circuit 35 detects the amplitude of the revolution frequency component instead of the vibration frequency component. Thus, the description is not repeated.

According to this embodiment, the plural coils 22A comprising the coils 22A and 22B are used as described above.

The magnetic field generated by a motor magnet (not shown) of the motor 17 is also applied to the coils 22. The magnitude of the magnetic field changes with the distance from the motor magnet. When each coil 22 is revolved, the magnitude of the magnetic field applied from the motor magnet to the coil 22 changes. According to the Faraday's electromagnetic induction law, voltage is output from the coil 22. This voltage has no relation to the magnetic fluid. Thus, noise occurs due to the voltage when the magnetic fluid is detected.

The magnitude of the magnetic field generated by the motor magnet is exponentially attenuated in the vicinity. It is estimated that the gradient of the magnetic field attenuation is constant over a length of a few centimeters with respect to a position significantly distant from the motor magnet.

Thus, it is estimated that when the coils 22A and 22B are revolved, the magnitudes of the magnetic fields applied from the motor magnet to the coils 22A and 22B change to the same degrees, and the voltages output from the coils 22A and 22B are equal.

As seen in the above-description, the effects of the motor magnet can be eliminated by subtraction of the outputs of the coils 22A and 22B.

Moreover, a resin is filled into the spaces existing in the body 23 of the detection unit 7, and is hardened, so that the plural coils 22 (22A and 22B), the exciting magnet 21, and the pre-amplification portion 24 are fixed. That is, the coils 22A and 22B are arranged in parallel, and in this sate, the resin is filled and hardened. Although the detection unit 7 is revolved with the revolution member 214, the relative directions and the relative positions of the coils 22A and 22B are prevented from changing.

In this case, it is assumed that the magnetic force lines of the terrestrial magnetism extend perpendicularly across the coils 22 (not shown).

The coils 22A and 22B are relatively moved, due to the revolution of the detection unit 7. Thus, their relative directions and positions are changed. Then, the magnetic force lines of the terrestrial magnetism extending across the coils 22A and 22B change. Accordingly, even if the subtraction is carried out on the outputs of the coils 22A and 22B, the magnetic noise, generated by the resultant magnetic field formed of the magnetic fields by the motor magnet and the terrestrial magnetism as described above, cannot be eliminated.

However, according to the distal-end revolution portion 210 shown in Fig. 57, the directions and the positions of the coils 22A and 22B are prevented from relatively changing, although the detection unit 7 is revolved accompanying the revolution of the revolution member 214, as described above. Therefore, the magnetic force lines of the terrestrial magnetism extending across the coils 22A and 22B do not change.

On the other hand, according to the distal-end revolution portion 210 shown in Fig. 58, the magnetic force lines of the terrestrial magnetism extending through the coils 22A and 22B change to the same degree. Therefore, the changes of the magnetic force lines of the terrestrial magnetism extending across the coils 22A and 22B can be cancelled out by subtraction of the outputs from the coils 22A and 22B.

Thus, according to this embodiment, the magnetic noise occurring due to the resultant magnetic field formed from the magnetic field by the motor magnet and that by the terrestrial magnetism, as described above, can be eliminated by subtraction of the outputs of the coils 22A and 22B.

The magnetic fluid detection device 201 having the above-described structure is applied to detect the magnetic fluid 6 staying in the sentinel lymph node 5 of a subject to identify the sentinel lymph node 5.

First, an operator punctures the lower layer of a lesion of the subject with a puncture needle (not shown), and infuses the magnetic fluid 6 locally in the vicinity of the lesion. Then, the magnetic fluid 6 infused in the vicinity of the lesion is moved from the infusion position to a lymph vessel, reaches the sentinel lymph node 5 five or fifteen minutes after the infusion, and stays in the sentinel lymph node 5.

Then, the operator surgically inserts the probe 2J of the magnetic fluid detection device 201 into an intracavity, e.g., via a trocar (not shown), or is placed on the surface of the subject body from the outside of the body. The operator detects the magnetic fluid 6 staying in the sentinel lymph node 5 while the operator moves the distal end of the probe 2J in the vicinity of the lesion of the patient.

At this time, in the probe 2J, the motor 17 is controlled and driven by the motor control circuit 36 of the control unit 4, and the rotation of the motor 17 is transmitted to the revolution member 214.

Then, in the probe 2J, the distal-end revolution portion 210 is revolved around its longitudinal axis by the revolution of the revolution member 214 around its longitudinal axis. Then, the exciting magnet 21 of the detection unit 7 is revolved around the longitudinal axis of the unit 7, and thereby, the probe 2J generates an AC magnetic field depending on the revolution frequency.

When the magnetic fluid 6 exists in the vicinity of the lesion of the patient, the AC magnetic field generated by the exciting magnet 21 excites the magnetic fluid 6 via the space in the vicinity of the probe. Then, the AC magnetic field is attracted or repelled in the vicinity of the magnetic fluid 6, so that the magnetic field distribution is locally distorted, and thus, the spatial gradient (magnetic flux density) of the magnetic field distribution changes. This local distortion of the magnetic field distribution (the change of the magnetic flux density), occurring due to the magnetic fluid 6, is detected by the plural coils 22 (22A and 22B).

Then, the coils 22A and 22B can detect a local distortion in magnetic field distribution (spatial magnetic gradient), occurring due to the magnetic fluid 6, while the detection is not affected by the exciting magnetic field (AC magnetic field). The outputs from the coils 24A and 24B are amplified by the preamplifiers 24A and 24B, and are transmitted to the line driver 26.

In the line driver 26, the subtracter 27 carries out the subtraction of the outputs from the coils 22A and 22B, and the difference is amplified by the amplifier 28 and transmitted to the control unit 4.

In this case, in the detection unit 7, together with the exciting magnet 21 and the plural coils 22 (22A and 22B), the preamplification portion 24 is revolved around its longitudinal axis, accompanying the revolution of the revolution member 214 around its longitudinal axis. Thus, the lead wires between the plural coils 22 (22A and 22B) and the pre-amplification portion 24 are not relatively revolved, as described above. The effects of changes in the contact resistances or the like can be eliminated.

The lead wires between the pre-amplification portion 24 and the line driver 26 are vibrated. However, the pre-amplification portion 24 and the line driver 26 are electrically connected to each other via the slip ring 229, as described above. Moreover, the fine outputs from the coils 22 are amplified in the pre-amplification portion 24. Thus, even if the signals are changed by changes in the contact resistances or the like, the changing degrees of the signals are very small compared to the magnitudes of the signals after the amplification is carried out. The effects of the changes of the signals are negligible. Thus, noise can be prevented from being generated due to the revolution of the coils 22 (22A and 22B) and the exciting magnet 21.

In the control unit 4, the higher harmonic component of the output signal received by the line receiver 31 is eliminated therefrom by LPF 32, and the amplitude component is taken out. The amplitude component is taken out. The amplitude component taken out is amplified by the amplifier 33, and is A/D converted by the A/D converter 34.

In the control unit 4, the digital signal processing circuit 35 carries out digital-signal-processing such as high speed Fourier transformation or the like of the outputs from the plural coils 22 (22A and 22B) (the digital signal from the A/D converter 34), based on the pulse signal from the motor control circuit 36, thereby to detect the amplitude of the revolution frequency component. Thus, the display 8 and the speaker 9 are driven in response to the amplitude of the detected signal.

The display 8 and the speaker 9 operate in the same manner as described in the first embodiment to inform the operator.

The magnetic fluid detection device 201 detects the local distortion of the magnetic field distribution generated by the magnetic fluid 6 by revolving the detection unit 7 with the revolution portion. Therefore, in the magnetic fluid detection device 201, the detection unit 7 can be easily revolved, and the revolution speed can be enhanced. Thus, the detection sensitivity for the magnetic fluid 6 is higher than that obtained when the detection unit 7 is vibrated.

Thus, the magnetic fluid detection device 201 of this embodiment can accurately detect the position of the magnetic fluid 6 staying in the sentinel lymph node 5 to identify the position of the sentinel lymph node 5 without being affected by the magnetic noise of the terrestrial magnetism or the like.

Sixth Embodiment

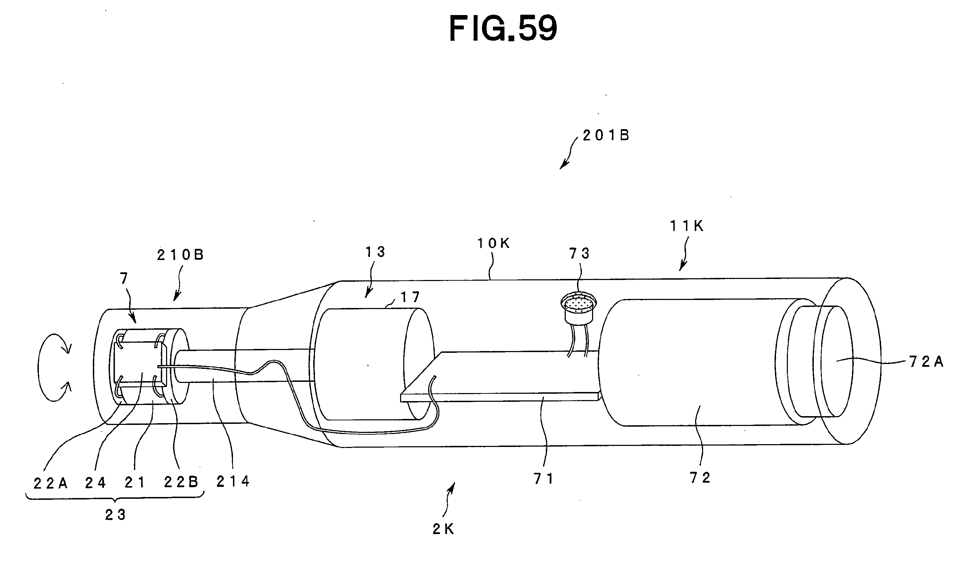

Figs. 59 to 60 show a magnetic fluid detection device according to a sixth embodiment of the present invention.

According to the fifth embodiment, the probe 2J and the control unit 4 are formed as separate pieces. According to the sixth embodiment, a control unit is contained in a probe. The other configuration is the same as that of the fifth embodiment. Thus, the description is not repeated. In the sixth embodiment, the same components as those of the fifth embodiment are designated by the same reference numerals.

In the magnetic fluid detection device 201B according to the sixth embodiment, a probe 2K contains the control unit 4 as shown in Fig. 59.

The body 11K of the probe 2K is provided with the control substrate 71 on the back side of the revolution-driving unit 213B. The battery 72 is provided on the back side of the control substrate 71.

The control substrate 71 is provided with LED 73 as a display. The LED 72 is connected to the control substrate 71. The battery 72 may be charged with electromotive power from the charging coil 72A.

The sheath 10K of the probe 2K is formed so as to be transparent. Thus, the light-emitting state of the LED 73 can be seen through the probe sheath 10K. The other configuration is the same as that of the fifth embodiment and the description thereof is omitted.

In the probe 2K, the motor 17 is revolved under control by a control circuit on the control substrate 71. The revolution motion is transmitted to the revolution member 214, and thus, the distal-end revolution portion 210B is revolved around its longitudinal axis.

Thus, the magnetic fluid detection device 201B of the sixth embodiment has the same advantages as those of the fifth embodiment. In addition, the device comprises the probe 2K only. Hence, the size can be educed, and the manipulation property is superior.

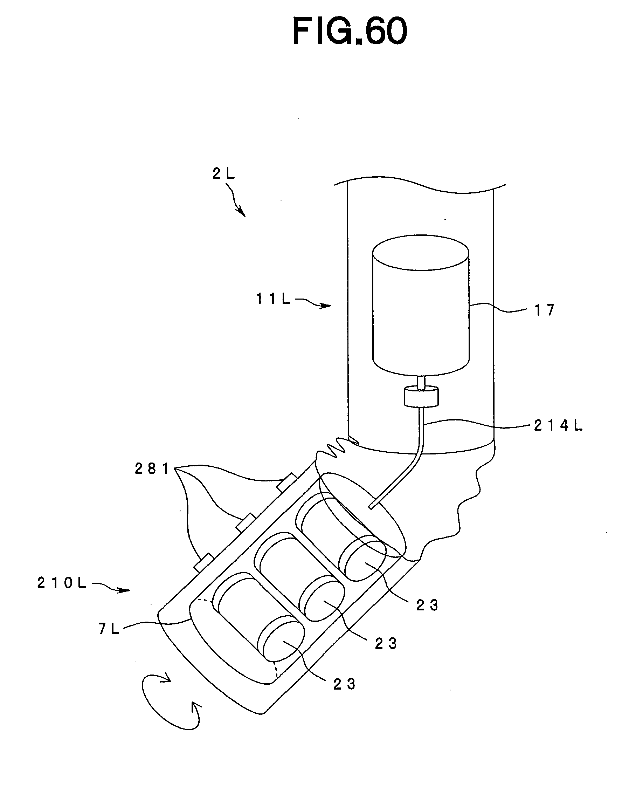

The probe may contain plural detection unit bodies 23 as shown in Fig. 60.

As shown in Fig. 60, the distal-end revolution portion 210L of the probe 2L contains freely revolutionarily provided detection unit 7 comprising plural detection unit bodies 23 (three detection unit bodies 23 in shown Fig. 60).

The revolution member 214L comprises a flexible shaft, and transmits the rotation of the motor 17 to the detection unit 7L, so that the detection unit 7L can be revolved around its longitudinal axis.

The distal-end revolution portion 210L is bendably connected to the probe body 11L via a bellows-connector. The bending direction can be controlled manually or by a bending operation wire.

LEDs 281 are provided for the distal-end revolution portion 210L, corresponding to the detection unit bodies 23. Thus, the position of the magnetic fluid can be more accurately detected. The other configuration is the same as that of the probe 2K, and thus, the description is not repeated.

As seen in the above-description, the probe 2L can detect the position of the magnetic fluid more accurately than the probe 2K.

In the fifth and sixth embodiments, the exciting electromagnet 90 may be used instead of the exciting magnet 21, which is a permanent magnet, used as the exciting unit.

Thus, it is not necessary to provide for the magnetic fluid detection device, a revolving mechanism. The structure of the magnetic fluid detection device is simple, the size can be reduced, and the manipulation property is superior.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

**Claims**

1. A magnetic fluid detection device (1) useful for detection of a magnetic fluid staying in a subject comprising:

    an exciting unit for generating an exciting magnetic field to excite a magnetic fluid (6) staying in a subject;
    a coil (22) for detecting a local distortion in magnetic field distribution occurring due to the magnetic fluid excited with the exciting magnetic field generated by the exciting unit; and
    a control unit (4) for signal-processing an output from the coil and informing the resultant signal magnitude.

2. A magnetic fluid detection device according to Claim 1, further comprising a driving unit for vibrating or revolving the exciting unit and the coil;
    wherein the exciting unit generates an exciting magnetic field depending on the frequency of the vibration or the frequency of the revolution generated by driving of the driving unit, and the control unit detects the frequency of the vibration or the frequency of the revolution generated by driving of the driving unit, and signal-processing the output from the coil, based on the detected vibration frequency or revolution frequency.

3. A magnetic fluid detection device according to Claim 1, wherein the exciting unit comprises an electromagnet, and the control unit controls the electromagnet so that the electromagnet generates an exciting magnetic field depending on a driving frequency, and signal-processing the output from the coil, based on the driving frequency.

4. A magnetic fluid detection device according to Claim 1, further comprising:

    a detection unit body containing at least the coil and the exciting unit;
    a driving unit for vibrating or revolving the detection unit body; and
    a probe body containing the driving unit and the detection unit body;
    the magnetic fluid detection device further comprising a sheath comprising a probe sheath covering the probe body and a distal-end cover provided separately from the probe sheath.

5. A magnetic fluid detection device according to Claim 1, wherein the coil has an aperture smaller than a lymph node of the subject.

6. A magnetic fluid detection device according to Claim 2, wherein the magnetic fluid detection device has a preamplifier for amplifying the output from the coil, and the preamplifier is vibrated or revolved integrally with the exciting unit and the coil.

7. A magnetic fluid detection device according to Claim 2, wherein the magnetic fluid detection device comprises plural coils, and the control unit subtracts one from another output of the plural coils to eliminate noise.

8. A magnetic fluid detection device according to Claim 2, wherein at least the exciting unit and the coil are arranged in the distal-end portion of the magnetic fluid detection device, and a member mechanically connected to the driving unit to be vibrated or revolved is made of a non-magnetic material.

9. A magnetic fluid detection device according to Claim 2, wherein the driving unit comprises a motor, and the magnetic fluid detection device has a magnetic field eliminating portion for eliminating the effects of a magnetic field generated by a motor magnet of the motor.

10. A magnetic fluid detection device according to Claim 2, wherein the exciting unit comprises an exciting magnet, and the exciting magnet has a correcting coil wound around thereon so that an AC magnetic field is generated so as to be applied to the coil only, and thereby, noise occurring by the positional shift of the coil when the driving unit id driven is eliminated.

11. A magnetic fluid detection device according to Claim 3, wherein the magnetic fluid detection device comprises plural coils, and the control unit eliminates noise by subtracting one from another output of the plural coils.

12. A magnetic fluid detection device according to Claim 4, wherein the magnetic fluid detection device has a water-tight member provided in a connection portion between the probe sheath and the distal-end cover.

13. A magnetic fluid detection device according to Claim 4, wherein the detection unit body is arranged in the distal-end portion of the magnetic fluid detection device, and a member mechanically connected to the driving unit to be vibrated or revolved is made of a non-magnetic material.

14. A magnetic fluid detection device according to Claim 7, wherein the control unit controls and drives the driving unit, and detects a vibration frequency component or a revolution frequency component using the difference between the outputs from the at least two coils, based on the operation state of the driving unit.

15. A magnetic fluid detection device according to Claim 7, wherein a resin is filled and hardened so that the relative directions and the relative positions of the at least two coils are not changed by the driving of the driving unit.

16. A magnetic fluid detection device according to Claim 7, wherein the driving unit has a movement amount increased by swinging the exciting unit and the at least two coils, so that the movement speed increases.

17. A magnetic fluid detection device according to Claim 9, wherein the magnetic field eliminating portion comprises a correcting magnet for canceling out the magnetic field of the motor magnet.

18. A magnetic fluid detection device according to Claim 9, wherein the magnetic field eliminating portion causes the motor magnet and the exciting unit to have the same polarity arrangement directions, when the relative magnetic permeability of the magnetic fluid is less than 1, and causes the motor magnet and the exciting unit to have the opposite polarity arrangement directions, when the relative magnetic permeability of the magnetic fluid is more than 1.

19. A magnetic fluid detection device according to Claim 9, wherein the magnetic field eliminating portion comprises a flexible shaft for positioning the motor far from the exciting unit and the coil.

20. A magnetic fluid detection device useful for detection of a magnetic fluid staying in a subject comprising:

a probe (2) comprising an exciting unit for generating an exciting magnetic field to excite a magnetic fluid staying in a subject, and a coil (22) for detecting a local distortion in magnetic field distribution occurring due to the magnetic fluid excited with the exciting magnetic field generated by the exciting unit; and
a control unit (4) for signal-processing an output from the coil provided in the probe (2) and informing the resultant signal magnitude.

21. A magnetic fluid detection device (1) useful for detection of a magnetic fluid staying in a subject comprising:

a probe (2) comprising an exciting unit for generating an exciting magnetic field to excite a magnetic fluid staying in a subject, a coil (22) for detecting a local distortion in magnetic field distribution occurring due to the magnetic fluid excited with the exciting magnetic field generated by the exciting unit, and a driving unit (13) for integrally vibrating or revolving the exciting unit and the coil; and
a control unit (4) for controlling and driving the driving unit (13) provided in the probe, signal-processing an output from the coil (22) and informing the resultant signal magnitude.

# FIG.1

# FIG.2

# FIG.3

EP 1 541 083 A2

# FIG.4

TO CONTROL UNIT

13

15

17

26

14

23

24A

N

S

21

22

7

# FIG.5

TO CONTROL UNIT

13

17

26

14

23

24A

N

S

21

22

7

# FIG.6

EP 1 541 083 A2

# FIG.7

MOTOR DRIVE SIGNAL

MOTOR CONTROL CIRCUIT  *36*

PULSE SIGNAL

SERVO SIGNAL

OUTPUT SIGNAL FROM LINE DRIVER

LINE RECEIVER  *31*

*37*

LPF  *32b*

DC AMPLIFIER  *33b*

VCO  *38*

LED  *8*

SPEAKER

*9*

*4B*

EP 1 541 083 A2

# FIG.8

EP 1 541 083 A2

FIG.9

FIG.10

FIG.11

FIG.12

22

22a

5
6

6b

6a

22

22a

5
6

6a

22a

22

EP 1 541 083 A2

# FIG.13

# FIG.18

FIG.14

FIG.15

FIG.16

FIG.17

# FIG.19

SIGNAL
MAGNITUDE

C    e    B    d    A    DISTANCE

EXTENDED DETECTION
DISTANCE

# FIG.21

SIGNAL
MAGNITUDE

DISTANCE

# FIG.20

EP 1 541 083 A2

TO CONTROL UNIT

# FIG.22

TO LINE DRIVER

# FIG.23

SIGNAL
MAGNITUDE

fO          f1          FREQUENCY

EP 1 541 083 A2

# FIG.24

EP 1 541 083 A2

EP 1 541 083 A2

# FIG.25

TO LINE DRIVER

1C

66a

66b

66a

23

S    N

7

22        21        24A

14

15

17

# FIG.26

# FIG.27

38

# FIG.28

EP 1 541 083 A2

# FIG.29

# FIG.30

# FIG.31

EP 1 541 083 A2

TO LINE DRIVER

13G

23

S    N

7

22    21    24A

14

75

75a

15

76a

76

# FIG.32

13H

75

14

75a

15

77

# FIG.33

76

76a

# FIG.34

76

76a

EP 1 541 083 A2

**FIG.35**

POSITION OF VIBRATION ROD / TIME

**FIG.36**

SIGNAL MAGNITUDE / TIME

AveS1 AveN1  AveS2 AveN2  AveS3 AveN3

SO1=AveS1−AveN1

SO2=AveS2−AveN2

SO3=AveS3−AveN3

**FIG.37**

SIGNAL MAGNITUDE / TIME

SO1  SO2  SO3

# FIG.38

EP 1 541 083 A2

# FIG.39

EP 1 541 083 A2

FIG.40

# FIG.41

# FIG.42

| | | |
|---|---|---|
| POSITION OF COIL 22A | POSITION OF COIL 22B | POSITION OF MOTOR UNIT |

MAGNETIC FIELD STRENGTH BY MOTOR MAGNET

0.5~1cm

EP 1 541 083 A2

# FIG.43

# FIG.44

H(t)

22

TERRESTRIAL MAGNETISM

# FIG.46

22A          22B          TERRESTRIAL MAGNETISM

# FIG.45

22A          22B          TERRESTRIAL MAGNETISM

EP 1 541 083 A2

# FIG.47

EP 1 541 083 A2

# FIG.48

13H

17

74

75

14   75a

15

17a

# FIG.49

74

# FIG.50

# FIG.51

27

24A                    24B

ELECTROMAGNET
DRIVER                 93

94

22B

91  } 90
92

22A

5

# FIG.52

SIGNAL
MAGNITUDE

FREQUENCY

# FIG.53

SIGNAL
MAGNITUDE

FREQUENCY

# FIG.54

SIGNAL
MAGNITUDE

FREQUENCY

# FIG.55

# FIG.56

# FIG.57

EP 1 541 083 A2

# FIG.58

EP 1 541 083 A2

# FIG.59

# FIG.60

# FIG.61